# EUROPÄISCHE PATENTSCHRIFT

(11) **EP 1 042 273 B1**
(45) Veröffentlichungstag und Bekanntmachung des Hinweises auf die Patenterteilung: **07.05.2003**
(21) Anmeldenummer: 98966901.5
(22) Anmeldetag: 18.12.1998
(51) Int. Cl.: C07C 69/75, C07C 67/303, C07C 51/36, C07C 61/09, C08K 5/12

(54) **VERFAHREN ZUR HYDRIERUNG VON BENZOLPOLYCARBONSÄUREN ODER DERIVATEN DAVON UNTER VERWENDUNG EINES MAKROPOREN AUFWEISENDEN KATALYSATORS**
METHOD FOR HYDROGENATING BENZENE POLYCARBOXYLIC ACIDS OR DERIVATIVES THEREOF BY USING A CATALYST CONTAINING MACROPORES
PROCEDE D'HYDROGENATION D'ACIDES POLYCARBOXYLIQUES DE BENZENE OU DE LEURS DERIVES A L'AIDE D'UN CATALYSEUR A MACROPORES

(30) Priorität: 19.12.1997 DE 19756913; 16.07.1998 DE 19832088
(43) Veröffentlichungstag der Anmeldung: 11.10.2000
(60) Teilanmeldung: 03004347.5
(73) Patentinhaber: BASF AKTIENGESELLSCHAFT, 67056 Ludwigshafen (DE)
(72) Erfinder: BRUNNER, Melanie, D-67105 Schifferstadt (DE); BÖTTCHER, Arnd, D-67227 Frankenthal (DE); BREITSCHEIDEL, Boris, D-67117 Limburgerhof (DE); HALBRITTER, Klaus, D-69124 Heidelberg (DE); HENKELMANN, Jochem, D-68165 Mannheim (DE); THIL, Lucien, D-67117 Limburgerhof (DE); PINKOS, Rolf, D-67098 Bad Dürkheim (DE)
(74) Vertreter: Isenbruck, Günter, Dr.
(86) Internationale Anmeldenummer: EP9808346
(87) Internationale Veröffentlichungsnummer: WO99032427

(56) Entgegenhaltungen:
- EP-A- 0 603 825
- WO-A-97/21792
- DE-A- 2 823 165
- JP-A- 7 011 074
- JP-A- 7 173 342
- JP-A- 9 249 890
- US-A- 2 070 770
- US-A- 3 027 398
- US-A- 3 334 149
- US-A- 3 730 931
- US-A- 5 286 898
- DATABASE WPI Week 9512 Derwent Publications Ltd., London, GB; AN 95-085556 XP002100461 & JP 07 011074 A (NEW JAPAN CHEMICAL CO LTD), 13. Januar 1995
- DATABASE WPI Week 9503 Derwent Publications Ltd., London, GB; AN 95-019405 XP002100462 & JP 06 306252 A (NEW JAPAN CHEMICAL CO LTD), 1. November 1994
- SEARS, DARBY: "The technology of plastizisers", 1982, WILEY SONS, NEW YORK
- WILSON: 'plasticisers-principles and practice' INST. OF MATERIALS Seiten 2.1 - 2.3.2
- Flexol-Weichmacher CC-55, Union Carbide Chemicals Company, Nov. 1957

## Beschreibung

Die vorliegende Erfindung betrifft ein Verfahren zur Hydrierung von Benzolpolycarbonsäuren oder Derivaten davon, wie z.B. Estern und/oder Anhydriden, durch Inkontaktbringen einer oder mehrerer Benzolpolycarbonsäuren oder eines oder mehrerer Derivate davon mit einem Wasserstoff enthaltenden Gas in Gegenwart eines Makroporen aufweisenden Katalysators.

Ferner betrifft die vorliegende Erfindung auch ausgewählte Vertreter der erhaltenen Hydrierungsprodukte an sich, d.h. die entsprechenden Cyclohexanverbindungen, insbesondere Cyclohexandicarbonsäureester und Cyclohexantricarbonsäureester, insbesondere der mit dem erfindungsgemäßen Verfahren erhaltenen Cyclohexandicarbonsäureester und Cyclohexantricarbonsäureester. Außerdem betrifft die vorliegende Erfindung auch die Verwendung der erhaltenen Cyclohexanpolycarbonsäuren als Weichmacher in Kunststoffen.

In der US 5,286,898 und der US 5,319,129 wird Dimethylterephthalat an geträgerten Pd-Katalysatoren, die mit Ni, Pt und/oder Ru versetzt sind, bei Temperaturen ≥ 140 °C und einem Druck zwischen 50 und 170 bar zum entsprechenden Hexahydrodimethylterephthalat hydriert. In der DE-A 28 23 165 werden aromatische Carbonsäureester an geträgerten Ni-, Ru-, Rh-, und/ oder Pd-Katalysatoren zu den entsprechenden cycloaliphatischen Carbonsäure-estern bei 70 bis 250 °C und 30 bis 200 bar hydriert. In der US 3,027,398 wird die Hydrierung von Dimethylterephthalat an geträgerten Ru-Katalysatoren bei 110 bis 140 °C und 35 bis 105 bar beschrieben.

In der DE-A 28 23 165 wird ein Verfahren zur katalytischen Hydrierung von aromatischen Carbonsäureestern beschrieben, wobei als geeignete Katalysatoren Ruthenium, Rhodium, Palladium oder deren Mischungen aufgebracht auf einen Träger erwähnt werden. Insbesondere werden in der DE-A 28 23 165 Palladium-Katalysatoren beschrieben, die auf einen Träger aus mindestens 20 % Lithium-Aluminium-Spinell aufgebracht sind. Als aromatische Carbonsäureester können die Ester von aromatischen Mono-, Di-, Tri- und Tetracarbonsäuren eingesetzt werden.

Die US 3,334,149 beschreibt ein Verfahren zur Herstellung von 1,4-Cyclohexandimethanol, das die Umsetzung von Dialkylterephthalaten in Gegenwart eines Palladium-Katalysators umfasst. Als geeigneter Katalysator wird beispielsweise Palladium auf einem Träger genannt, wobei auf die genaue Struktur des Trägers nicht eingegangen wird. Auch die US 3,027,398 betrifft ein Hydrierverfahren zur Herstellung von Dimethyl-1,4-cyclohexandicarboxylaten. Die Hydrierung kann beispielsweise in Gegenwart von Ruthenium-Katalysatoren durchgeführt werden. In der US 2,070,770 wird ein Verfahren zur selektiven Hydrierung von aromatischen Estern in Gegenwart eines Hydrierungskatalysators beschrieben. Insbesondere wird in der US 2,070,770 die Hydrierung mittels NickelKatalysatoren erwähnt.

Die EP-A 0 603 825 betrifft ein Verfahren zur Herstellung von 1,4-Cyclohexandicarbonsäure durch Hydrierung von Terephthalsäure unter Verwendung eines geträgerten Palladium-Katalysators, wobei als Träger Aluminiumoxid, Siliciumdioxid oder Aktivkohle verwendet wird. Das dort beschriebene Verfahren ist insbesondere dadurch charakterisiert, daß die in einer ersten Stufe erhaltene 1,4-Cyclohexandicarbonsäure enthaltende Lösung mit Dampf in Kontakt gebracht wird und dadurch in dieser Lösung enthaltene Verunreinigungen extrahiert werden. Dieses Verfahren ist jedoch nur auf Säuren anwendbar, da bei der Anwendung auf Derivate, wie z.B. Ester, Anhydride, usw. die Gefahr von Hydrolyse besteht. Die Verwendung eines Makroporen aufweisenden Trägers wird in dieser Anmeldung mit keinem Wort erwähnt.

Bislang wurden als Weichmacher in Kunststoffen, wie z.B. PVC sehr häufig Phthalsäureestern, wie z.B. Dibutyl-, Dioctyl- oder Diisononylester der Phthalsäure verwendet, wie dies z.B. aus der FR-A 23 97 131 hervorgeht. Diesen wird jedoch seit kurzer Zeit nachgesagt, daß sie gesundheitlich nicht unbedenklich sind, sodaß ihre Verwendung in Kunststoffen zur Verwendung von z.B. Kinderspielzeug immer stärker in der Kritik steht und in einigen Ländern bereits verboten ist.

Die Verwendung von einigen Cyclohexan-1,2-dicarbonsäureestern als Weichmacher ist ebenfalls aus dem Stand der Technik bekannt. So sind die Verwendung von Cyclohexandicarbonsäuredimethyl oder -diethylestern (DE-A 28 23 165) und Cyclohexan-1,2-dicarbonsäuredi(2-ethylhexyl)ester (DE-A 12 63 296), als Weichmacher in Kunstoffen beschrieben.

Aus dem Stand der Technik (beispielsweise "The Technology of Plasticizers", J. Kern Sears, Joseph R. Darby, John Wiley & Sons, New York, 1982, 964 - 969, 864; "Plasticizers - Principles and Practice", Alan S. Wilson, Institute of Materials, Kapitel 2.1 bis 2.3.2 oder US 3,730,931) ist bekannt, dass Di-(2-ethylhexyl)cyclohexandicarbonsäureester als Weichmacher, beispielsweise für PVC, Verwendung findet, Auch ausgewählte Cyclohexandicarbonsäuredialkylester mit linearen oder verzweigten Alkylresten sind aus dem Stand der Technik bekannt. So offenbaren beispielsweise die WO 97/21792 oder die JP 09-249890 Schmiermittelzusammensetzungen, die Cyclohexandicarbonsäurediester enthalten können.

In JP 07-173342, JP 07-711074 oder JP 06-306252 werden Zusammensetzungen enthaltend ein Polyolefin sowie einen 1,2-Cyclohexandicarbonsäuredialkylester mit einem linearen oder verzweigten Alkylrest mit 6 bis 28 Kohlenstoffatomen offenbart.

Der vorliegenden Erfindung lag die primäre Aufgabe zugrunde, ein Verfahren zur Hydrierung von Benzolpolycarbonsäure(derivate)n, insbesondere Beozoldicarbonsäureestern unter Verwendung spezifischer Katalysatoren zur Verfügung zu stellen, mit deren Hilfe die entsprechenden kemhydrierten Derivate, insbesondere Cyclohexandicarbonsäureester mit sehr hoher Selektivität und Raum-Zeit-Ausbeute ohne signifikante Nebenreaktionen erhalten werden können.

Eine weitere Aufgabe der vorliegenden Erfindung lag in der Bereitstellung neuer Produkte, die durch die erfindungsgemäße Hydrierung von Benzolpolycarbonsäure(derivaten) erhältlich sind, und sich vorzugsweise zur Verwendung als Weichmacher in Kunststoffen eignen sollten.

Demgemäß betrifft die vorliegende Erfindung ein Verfahren zur Hydrierung einer Benzolpolycarbonsäure oder eines Derivats davon oder eines Gemischs aus zwei oder mehr davon durch Inkontaktbringen der Benzolpolycarbonsäure oder des Derivats davon oder des Gemischs aus zwei oder mehr davon mit einem Wasserstoff enthaltenden Gas in Gegenwart eines Katalysators, der als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfaßt, dadurch gekennzeichnet, daß der Träger Makroporen unit einem Poren durch messer oberhalb von 50 nm gemäß der Definition in Pure Applied Chemistry, 45, S. 79 (1976) aufweist, und
dass der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt, wobei 10 bis 50% des Porenvolumens des Trägers von Makroporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet werden, wobei sich die Summe der Anteile Porenvolumina zu 100% addiert,
mit der Maßgabe, daß
sofern Terephthalsäuredimethylester hydriert wird, die Hydrierung mit einem Katalysator, der
als Aktivmetall Ruthenium alleine oder zusammen mit mindestens einem Metall der I., VII oder VIII. Nebengruppe des Periodensystems, aufgebracht auf einem Träger, umfaßt, wobei der Träger einen mittleren Porendurchmesser von mindestens 50 nm und eine BET-Oberfläche von höchstens 30 m²/g aufweist und die Menge des Aktivmetalls 0,01 bis 30 Gew.- %, bezogen auf das Gesamtgewicht des Katalysator, beträgt, und das Verhältnis der Oberflächen des Aktivmetalls und des Katalysatorträgers kleiner 0,05 ist, und/oder
einem Katalysator, der als Aktivmetall Ruthenium alleine oder zusammen mit mindestens einem Metall der I., VII oder VIII. Nebengruppe des Periodensystems in
einer Menge von 0,01 bis 30 Gew.- %, bezogen auf das Gesamtgewicht des Katalysators, aufgebracht auf einem Träger, umfaßt, wobei 10 bis 50% des Porenvolumens des Trägers von Makroporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet werden, wobei sich die Summe der Anteile der Porenvolumina zu 100% addiert, ausgeschlossen ist.

In einer bevorzugten Ausführungsform betrifft die vorliegende Erfindung ein Verfahren zur Hydrierung einer Benzolpolycarbonsäure oder eines Derivats davon oder eines Gemischs aus zwei oder mehr davon, wobei der Katalysator als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfaßt, wobei der Träger einen mittleren Porendurchmesser von mindestens 50 nm und eine BET-Oberfläche von höchstens 30 m²/g aufweist und die Menge des Aktivmetalls 0,01 bis 30 Gew.-%, bezogen auf das Gesamtgewicht des Katalysators, beträgt (Katalysator 1).

Die Erfindung betrifft ein Verfahren, wobei der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt, wobei 10 bis 50% des Porenvolumens des Trägers von Makroporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet werden, wobei sich die Summe der Anteile der Porenvolumina zu 100% addiert (Katalysator 2).

In einer weiteren bevorzugten Ausführungsform betrifft die vorliegende Erfindung ein Verfahren, wie oben definiert, wobei der Katalysator (Katalysator 3) als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%. bezogen auf das Gesamtgewicht des Katalysators, aufgebracht auf einem Träger, umfaßt, wobei der Träger einen mittleren Porendurchmesser von mindestens 0,1 µm, und eine BET-Oberfläche von höchstens 15 m²/g aufweist. Als Träger können prinzipiell alle Träger eingesetzt werden, die Makroporen aufweisen, d.h. Träger, die ausschließlich Makroporen aufweisen sowie solche, die neben Makroporen auch Meso- und/oder Mikroporen enthalten.

Als Aktivmetall können prinzipiell alle Metalle der VIII. Nebengruppe des Periodensystems eingesetzt werden. Vorzugsweise werden als Aktivmetalle Platin, Rhodium, Palladium, Cobalt, Nickel oder Ruthenium oder ein Gemisch aus zwei oder mehr davon eingesetzt, wobei insbesondere Ruthenium als Aktivrnetall verwendet wird. Unter den ebenfalls verwendbaren Metallen der I. oder VII. oder aber der I. und der VII. Nebengruppe des Periodensystems, die ebenfalls allesamt prinzipiell verwendbar sind, werden vorzugsweise Kupfer und/oder Rhenium eingesetzt.

Die Begriffe *"Makroporen"* und *"Mesoporen"* werden im Rahmen der vorliegenden Erfindung so verwendet, wie sie in *Pure Appl. Chem., 45, S.* 79 *(1976)* definiert sind, nämlich als Poren, deren Durchmesser oberhalb von 50 nm (Makroporen) oder deren Durchmesser zwischen 2 nm und 50 nm liegt (Mesoporen).

Der Gehalt des Aktivmetalls beträgt 0,01 bis 30 Gew.-%, vorzugsweise ungefähr 0,01 bis ungefähr 5 Gew.-% und insbesondere ungefähr 0,1 bis ungefähr 5 Gew.-%, jeweils bezogen auf das Gesamtgewicht des verwendeten Katalysators, wobei die bei den im folgenden beschriebenen, vorzugsweise eingesetzten Katalysatoren 1 bis 3 vorzugsweise verwendeten Gehalte nochmals bei der Diskussion dieser Katalysatoren einzeln angegeben sind.

Der erfindungsgemäß verwendete Begriff *"Benzolpolycarbonsäure oder eines* *Derivats davon"* umfaßt alle Benzolpolycarbonsäuren an sich, wie z.B. Phthalsäure, Isophthalsäure, Terephthalsäure, Trimellitsäure, Trimesinsäure, Hemimellitsäure und Pyrromellitsäure und Derivate davon, wobei insbesondere Mono-, Di- und ggf. Tri- und Tetraester, insbesondere Alkylester, und Anhydride zu nennen sind. Die vorzugsweise eingesetzten Verbindungen werden untenstehend im Abschnitt "Die Verfahrensführung" nochmals kurz erläutert.

Im folgenden sollen nunmehr die vorzugsweise verwendeten Katalysatoren 1 bis 3 detailliert beschrieben werden. Dabei erfolgt die Beschreibung beispielhaft unter Bezugnahme auf die Verwendung von Ruthenium als Aktivmetall. Die untenstehenden Angaben sind auch auf die anderen verwendbaren Aktivmetalle, wie hierin definiert, übertragbar.

### KATALYSATOR 1

Die erfindungsgemäß verwendeten Katalysatoren 1 können technisch hergestellt werden durch Auftragen mindestens eines Metalls der VIII. Nebengruppe des Periodensystems und gegebenenfalls mindestens eines Metalls der I. oder VII. Nebengruppe des Periodensystems auf einem geeigneten Träger.

Die Auftragung kann durch Tränken des Trägers in wäßrigen Metallsalzlösungen, wie z.B. wäßrigen Rutheniumsalzlösungen, durch Aufsprühen entsprechender Metallsalzlösungen auf den Träger oder durch andere geeignete Verfahren erreicht werden. Als Metallsalze der I., VII. oder VIII. Nebengruppe des Periodensystems eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die neben dem Metall der VIII. Nebengruppe des Periodensystems noch weitere Metalle als Aktivmetall auf dem Träger aufgetragen enthalten, können die Metallsalze bzw. Metallsalzlösungen gleichzeitig oder nacheinander aufgebracht werden.

Die mit der Metallsalzlösung beschichteten bzw. getränkten Träger werden anschließend, vorzugsweise bei Temperaturen von 100 bis 150 °C, getrocknet und wahlweise bei Temperaturen von 200 bis 600 °C, vorzugsweise von 350 bis 450 °C calciniert. Bei getrennter Auftränkung wird der Katalysator nach jedem Tränkschritt getrocknet und wahlweise calciniert, wie oben beschrieben. Die Reihenfolge, in der die Aktivkomponenten aufgetränkt werden, ist dabei frei wählbar.

Anschließend werden die beschichteten und getrockneten sowie wahlweise calcinierten Träger durch Behandlung in einem Gasstrom, der freien Wasserstoff enthält, bei Temperaturen von ungefähr 30 bis ungefähr 600 °C, vorzugsweise von ungefähr 150 bis ungefähr 450 °C aktiviert. Vorzugsweise besteht der Gasstrom aus 50 bis 100 Vol.-% H₂ und 0 bis 50 Vol.-% N₂.

Die Metallsalzlösung oder -lösungen werden in einer solchen Menge auf den oder die Träger aufgebracht, daß der Gesamtgehalt an Aktivmetall, jeweils bezogen auf das Gesamtgewicht des Katalysators, ungefähr 0,01 bis ungefähr 30 Gew.-%, vorzugsweise ungefähr 0,01 bis ungefähr 5 Gew.-%, weiter bevorzugt ungefähr 0,01 bis ungefähr 1 Gew.-%, und insbesondere ungefähr 0,05 bis ungefähr 1 Gew.-% beträgt.

Die Metalloberfläche auf dem Katalysator 1 beträgt dabei insgesamt vorzugsweise ungefähr 0,01 bis ungefähr 10 m²/g, weiter bevorzugt ungefähr 0,05 bis ungefähr 5 m²/g und insbesondere ungefähr 0,05 bis ungefähr 3 m²/g des Katalysators. Die Metalloberfläche wird mittels der von J. Lemaitre et al. in "*Characterization of Heterogeneous Catalysts*", Hrsg. Francis Delanney, Marcel Dekker, New York 1984, S. 310 - 324, beschriebenen Chemisorptionsverfahren bestimmt.

Im erfindungsgemäß verwendeten Katalysator 1 beträgt das Verhältnis der Oberflächen des/der Aktivmetalls/.-metalle und des Katalysatorträgers vorzugsweise weniger als ungefähr 0,05, wobei der untere Grenzwert bei ungefähr 0,0005 liegt.

Die zur Herstellung der erfindungsgemäß verwendeten Katalysatoren verwendbaren Trägermaterialien sind solche, die makroporös sind und einen mittleren Porendurchmesser von mindestens ungefähr 50 nm, vorzugsweise mindestens ungefähr 100 nm, insbesondere mindestens ungefähr 500 nm aufweisen und deren Oberfläche nach BET bei höchstens ungefähr 30 m²/g, vorzugsweise höchstens ungefähr 15 m²/g, weiter bevorzugt höchstens ungefähr 10 m²/g, insbesondere höchstens ungefähr 5 m²/g und weiter bevorzugt höchstens ungefähr 3 m²/g liegt. Der mittlere Porendurchmesser des Trägers beträgt vorzugsweise ungefähr 100 nm bis ungefähr 200 µm, weiter bevorzugt ungefähr 500 nm bis ungefähr 50 µm. Die Oberfläche des Trägers beträgt vorzugsweise ungefähr 0,2 bis ungefähr 15 m²/g, weiter bevorzugt ungefähr 0,5 bis ungefähr 10 m²/g, insbesondere ungefähr 0,5 bis ungefähr 5 m²/g und weiter bevorzugt ungefähr 0,5 bis ungefähr 3 m²/g.

Die Oberfläche des Trägers wird bestimmt nach dem BET-Verfahren durch N₂-Adsorption, insbesondere nach DIN 66131. Die Bestimmung des mittleren Porendurchmesser und der Porengrößenverteilung erfolgt durch Hg-Porosimetrie, insbesondere nach DIN 66133.

Vorzugsweise kann die Porengrößenverteilung des Trägers annähernd bimodal sein, wobei die Porendurchmesserverteilung mit Maxima bei etwa 600 nm und etwa 20 µm bei der bimodalen Verteilung eine spezielle Ausführungsform der Erfindung darstellt.

Weiter bevorzugt ist ein Träger mit einer Oberfläche von 1,75 m²/g, der diese bimodale Verteilung des Porendurchmessers aufweist. Das Porenvolumen dieses bevorzugten Trägers beträgt vorzugsweise etwa 0,53 ml/g.

Als makroporöses Trägermaterial verwendbar sind beispielsweise Makroporen aufweisende Aktivkohle, Siliciumcarbid, Aluminiumoxid, Siliciurndioxid, Titandioxid, Zirkoniumdioxid, Magnesiumoxid, Zinkoxid oder Gemische aus zwei oder mehr davon, wobei Aluminiumoxid und Zirkoniumdioxid vorzugsweise verwendet werden.

Weitere Details bezüglich Katalysator 1 bzw. zu seiner Herstellung sind der DE-A 196 24 484.6 zu entnehmen, deren diesbezüglicher Inhalt durch Bezugnahme vollständig in die vorliegende Anmeldung einbezogen wird.

### KATALYSATOR 2

Die erfindungsgemäß verwendeten Katalysatoren 2 enthalten ein oder mehrere Metalle der VIII. Nebengruppe des Periodensystems als Aktivkomponente(n) auf einem Träger, wie hierin definiert. Bevorzugt werden Ruthenium, Palladium und/oder Rhodium als Aktivkomponente(n) verwendet.

Die erfindungsgemäß verwendeten Katalysatoren 2 können technisch hergestellt werden durch Auftragen mindestens eines Aktivmetalls der VIII. Nebengruppe des Periodensystems, vorzugsweise Ruthenium oder Palladium und gegebenenfalls mindestens eines Metalls der I. oder VII. Nebengruppe des Periodensystems auf einem geeigneten Träger. Die Auftragung kann durch Tränken des Trägers in wäßrigen Metallsalzlösungen, wie z.B. Ruthenium- oder Palladiumsalzlösungen, durch Aufsprühen entsprechender Metallsalzlösungen auf den Träger oder durch andere geeignete Verfahren erreicht werden. Als Metallsalze zur Herstellung der Metallsalzlösungen eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die mehrere Aktivmetalle auf den Träger aufgetragen enthalten, können die Metallsalze bzw. Metallsalzlösungen gleichzeitig oder nacheinander aufgebracht werden.

Die mit der Metallsalzlösung beschichteten bzw. getränkten Träger werden anschließend getrocknet, wobei Temperaturen von 100 bis 150 °C bevorzugt sind. Wahlweise können diese Träger bei Temperaturen von 200 bis 600 °C, vorzugsweise von 350 bis 450 °C calciniert werden. Anschließend werden die beschichteten Träger durch Behandlung in einem Gasstrom, der freien Wasserstoff enthält, bei Temperaturen von 30 bis 600 °C, vorzugsweise von 100 bis 450 °C und insbesondere von 100 bis 300 °C aktiviert. Der Gasstrom besteht vorzugsweise aus 50 bis 100 Vol.-% H₂ und 0 bis 50 Vol.-% N₂.

Werden auf die Träger mehrere Aktivmetalle aufgetragen und erfolgt das Auftragen nacheinander, so kann der Träger nach jedem Auftragen bzw. Tränken bei Temperaturen von 100 bis 150 °C getrocknet werden und wahlweise bei Temperaturen von 200 bis 600 °C calciniert werden. Dabei kann die Reihenfolge, in der die Metallsalzlösung aufgetragen oder aufgetränkt wird, beliebig gewählt werden.

Die Metallsalzlösung wird in einer solchen Menge auf den/die Träger aufgebracht, daß der Gehalt an Aktivmetall 0,01 bis 30 Gew.-%, vorzugsweise 0,01 bis 10 Gew.-%, weiter bevorzugt 0,01 bis 5 Gew.-%, und insbesondere 0,3 bis 1 Gew.-%, bezogen auf das Gesamtgewicht des Katalysators, beträgt.

Die Metalloberfläche auf dem Katalysator beträgt insgesamt vorzugsweise 0,01 bis 10 m²/g, besonders bevorzugt 0,05 bis 5 m²/g und weiter bevorzugt 0,05 bis 3 m²/g des Katalysators. Die Metalloberfläche wurde durch das Chemisorptionsverfahren gemessen, wie es in J, Lemaitre et al., "*Characterization of Heterogeneous Catatysts*", Hrsg. Francis Delanney, Marcel Dekker, New York (1984), S. 310 - 324, beschrieben ist.

Im erfindungsgemäß verwendeten Katalysator 2 beträgt das Verhältnis der Oberflächen des mindestens einen Aktivmetalls und des Katalysatorträgers weniger als ungefähr 0,3, vorzugsweise weniger als ungefähr 0,1 und insbesondere ungefähr 0,05 oder weniger, wobei der untere Grenzwert bei ungefähr 0,0005 liegt.

Die zur Herstellung der erfindungsgemäß verwendeten Katalysatoren 2 verwendbaren Trägermaterialien besitzen Makroporen und Mesoporen.

Dabei weisen die erfindungsgemäß verwendbaren Träger eine Porenverteilung auf, dergemäß ungefähr 5 bis ungefähr 50%, vorzugsweise ungefähr 10 bis ungefähr 45%, weiter bevorzugt ungefähr 10 bis ungefähr 30 und insbesondere ungefähr 15 bis ungefähr 25% des Porenvolumens von Makroporen mit Porendurchmessern im Bereich von ungefähr 50 nm bis ungefähr 10.000 nm und ungefähr 50 bis ungefähr 95%, vorzugsweise ungefähr 55 bis ungefähr 90%, weiter bevorzugt ungefähr 70 bis ungefähr 90% und insbesondere ungefähr 75 bis ungefähr 85% des Porenvolumens von Mesoporen mit einem Porendurchmesser von ungefähr 2 bis ungefähr 50 nm gebildet werden, wobei sich jeweils die Summe der Anteile der Porenvolumina zu 100% addiert.

Das Gesamtporenvolumen der erfindungsgemäß verwendeten Träger beträgt ungefähr 0,05 bis 1,5 cm³/g, vorzugsweise 0,1 bis 1,2 cm³/g und insbesondere ungefähr 0,3 bis 1,0 cm³/g. Der mittlere Porendurchmesser der erfindungsgemäß verwendeten Träger beträgt ungefähr 5 bis 20 nm, vorzugsweise ungefähr 8 bis ungefähr 15 nm und insbesondere ungefähr 9 bis ungefähr 12 nm.

Vorzugsweise beträgt die Oberfläche des Trägers ungefähr 50 bis ungefähr 500 m²/g, weiter bevorzugt ungefähr 200 bis ungefähr 350 m²/g und insbesondere ungefähr 250 bis ungefähr 300 m²/g des Trägers.

Die Oberfläche des Trägers wird nach dem BET-Verfahren durch N₂-Adsorption, insbesondere nach DIN 66131, bestimmt. Die Bestimmung des mittleren Porendurchmesser und der Größenverteilung erfolgt durch Hg-Porosimetrie, insbesondere nach DIN 66133.

Obwohl prinzipiell alle bei der Katalysatorherstellung bekannten Trägermaterialien, d.h. die die oben definierte Porengrößenverteilung aufweisen, eingesetzt werden können, werden vorzugsweise Makroporen aufweisende Aktivkohle, Siliciumcarbid, Aluminiumoxid, Siliciumdioxid, Titandioxid, Zirkoniumdioxid, Magnesiumoxid, Zinkoxid oder deren Gemische, weiter bevorzugt Aluminiumoxid und Zirkoniumdioxid, eingesetzt.

Weitere Details bezüglich Katalysator 2 bzw. zu seiner Herstellung sind der DE-A 196 24 485.4 zu entnehmen, deren diesbezüglicher Inhalt durch Bezugnahme vollständig in die vorliegende Anmeldung einbezogen wird.

### KATALYSATOR 3

Die erfindungsgemäß verwendeten Katalysatoren 3 können technisch hergestellt werden durch Aurtragen eines Aktivmetalls der VIII. Nebengruppe des Periodensystems und gegebenenfalls mindestens eines Metalls der I. oder VII. Nebengruppe des Periodensystems auf einen geeigneten Träger. Die Auftragung kann durch Tränken des Trägers in wäßrigen Metallsalzlösungen, wie z.B. Rutheniumsalzlösungen, durch Aufsprühen entsprechender Metallsalzlösungen auf den Träger oder durch andere geeignete Verfahren erreicht werden. Als Rutheniumsalze zur Herstellung der Rutheniumsalzlösungen wie auch als Metallsalze der I., VII. oder VIII. Nebengruppe eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, bevorzugt sind dabei die Nitrate und Nitrosylnitrate.

Bei Katalysatoren, die mehrere Metalle auf den Träger aufgetragen enthalten, können die Metallsalze bzw. Metallsalzlösungen gleichzeitig oder nacheinander aufgebracht werden.

Die mit der Rutheniumsalz- bzw. Metallsalzlösung beschichteten bzw. getränkten Träger werden sodann getrocknet, vorzugsweise bei Temperaturen von 100 bis 150 °C, und wahlweise bei Temperaturen von 200 bis 600 °C calciniert.

Darauffolgend werden die beschichteten Träger aktiviert durch Behandlung der beschichteten Träger in einem Gasstrom, der freien Wasserstoff enthält, bei Temperaturen von 30 bis 600 °C, vorzugsweise von 150 bis 450 °C. Der Gasstrom besteht vorzugsweise aus 50 bis 100 Vol-% H₂ und 0 bis 50 Vol-% N₂.

Werden auf die Träger neben dem Aktivmetall der VIII. Nebengruppe des Periodensystems Metalle der I. oder VII. Nebengruppe aufgetragen und erfolgt das Auftragen nacheinander, so kann der Träger nach jedem Auftragen bzw. Tränken bei Temperaturen von 100 bis 150 °C getrocknet werden und wahlweise bei Temperaturen von 200 bis 600 °C calciniert werden. Dabei kann die Reihenfolge, in der die Metallsalzlösungen aufgetragen oder aufgetränkt werden, beliebig gewählt werden.

Die Metallsalzlösung wird in einer solchen Menge auf den oder die Träger aufgebracht, daß 0,01 bis 30 Gew.-%, bezogen auf das Gesamtgewicht des Katalysators, an Aktivmetall auf den Träger aufgebracht vorliegen. Vorzugsweise beträgt diese Menge 0,2 bis 15 Gew.-%, besonders bevorzugt etwa 0,5 Gew.-%.

Die Metalloberfläche auf dem Katalysator 3 beträgt insgesamt vorzugsweise 0,01 bis 10 m²/g, besonders bevorzugt 0,05 bis 5 m²/g, insbesondere 0,05 bis 3 m² pro g des Katalysators.

Die zur Herstellung der erfindungsgemäß verwendeten Katalysatoren 3 verwendbaren Trägermaterialien sind vorzugsweise solche, die makroporös sind und einen mittleren Porendurchmesser von mindestens 0,1 µm, vorzugsweise mindestens 0,5 µm, und eine Oberfläche von höchstens 15 m²/g aufweisen, vorzugsweise höchstens 10 m²/g, besonders bevorzugt höchstens 5 m²/g, insbesondere höchstens 3 m²/g. Bevorzugt liegt der mittlere Porendurchmesser des Trägers in einem Bereich von 0,1 bis 200 µm, insbesondere von 0,5 bis 50 µm. Bevorzugt beträgt die Oberfläche des Trägers 0,2 bis 15 m²/g, besonders bevorzugt 0,5 bis 10 m²/g, insbesondere 0,5 bis 5 m²/g, speziell 0,5 bis 3 m²/g des Trägers.

Die Oberfläche des Trägers wird bestimmt nach dem BET-Verfahren durch N2-Adsorption, insbesondere nach DIN 66131. Die Bestimmung des mittleren Porendurchmessers und der Porengrößenverteilung erfolgte durch Hg-Porosimetrie, insbesondere nach DIN 66133. Vorzugsweise kann die Porengrößenverteilung des Trägers annähernd bimodal sein, wobei die Porendurchmesserverteilung mit Maxima bei etwa 0,6 µm und etwa 20 µm bei der bimodalen Verteilung eine spezielle Ausführungsform der Erfindung darstellt.

Besonders bevorzugt ist ein Träger mit einer Oberfläche von etwa 1,75 m²/g, der diese bimodale Verteilung des Porendurchmessers aufweist. Das Porenvolumen dieses bevorzugten Trägers beträgt vorzugsweise etwa 0,53 ml/g.

Als makroporöses Trägermaterial verwendbar sind beispielsweise Makroporen aufweisende Aktivkohle, Siliciumcarbid, Aluminiumoxid, Siliciurndioxid, Titandioxid, Zirkoniumdioxid, Magnesiumoxid, Zinkoxid oder deren Gemische. Bevorzugt sind Aluminiumoxid und Zirkoniumdioxid.

Weitere Details bezüglich Katalysator 3 bzw. zu seiner Herstellung sind der DE-A 196 04 791.9 zu entnehmen, deren diesbezüglicher Inhalt durch Bezugnahme vollständig in die vorliegende Anmeldung einbezogen wird.

### DIE VERFAHRENSFÜHRUNG

Im Rahmen des erfindungsgemäßen Verfahrens wird die Hydrierung im allgemeinen bei einer Temperatur von ungefähr 50 bis 250 °C, vorzugsweise ungefähr 70 bis 220 °C durchgeführt. Die dabei verwendeten Drücke liegen in der Regel bei oberhalb von 10 bar, vorzugsweise ungefähr 20 bis ungefähr 300 bar.

Das erfindungsgemäße Verfahren kann entweder kontinuierlich oder diskontinuierlich durchgeführt werden, wobei die kontinuierliche Verfahrensdurchführung bevorzugt ist.

Bei der kontinuierlichen Verfahrensführung beträgt die Menge der (des) zur Hydrierung vorgesehenen Benzolpolycarbonsäure(esters) bzw. des Gemischs aus zwei oder mehr davon vorzugsweise ungefähr 0,05 bis ungefähr 3 kg pro Liter Katalysator pro Stunde, weiter bevorzugt ungefähr 0,1 bis ungefähr 1 kg pro Liter Katalysator pro Stunde.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet.

Die erfindungsgemäße Hydrierung kann in Ab- oder Anwesenheit eines Lösungsoder Verdünnungsmittels durchgeführt werden, d.h. es ist nicht erforderlich, die Hydrierung in Lösung durchzuführen.

Vorzugsweise wird jedoch ein Lösungs- oder Verdünnungsmittel eingesetzt. Als Lösungs- oder Verdünnungsmittel kann jedes geeignete Lösungsmittel- oder Verdünnungsmittel eingesetzt werden. Die Auswahl ist dabei nicht kritisch, solange das eingesetzte Lösungs- oder Verdünnungsmittel in der Lage ist, mit der (dem) zu hydrierenden Benzoldicarbonsäure(ester) eine homogene Lösung zu bilden. Beispielsweise können die Lösungs- oder Verdünnungsmittel auch Wasser enthalten.

### Beispiele geeigneter Lösungs- oder Verdünnungsmittel schließen die folgenden ein:

Geradkettige oder cyclische Ether, wie beispielsweise Tetrahydrofuran oder Dioxan, sowie aliphatische Alkohole, in denen der Alkylrest vorzugsweise 1 bis 10 Kohlenstoffatome, insbesondere 3 bis 6 Kohlenstoffatome aufweist.

Beispiele bevorzugt verwendbarer Alkohole sind i-Propanol, n-Butanol, i-Butanol und n-Hexanol.

Gemische dieser oder anderer Lösungs- oder Verdünnungsmittel können ebenfalls verwendet werden.

Die Menge des eingesetzten Lösungs- oder Verdünnungsmittels ist nicht in besonderer Weise beschränkt und kann je nach Bedarf frei gewählt werden, wobei jedoch solche Mengen bevorzugt sind, die zu einer 10 bis 70 Gew.-%igen Lösung der (des) zur Hydrierung vorgesehenen Benzoldicarbonsäure(esters) führen.

Besonders bevorzugt wird im Rahmen des erfindungsgemäßen Verfahrens das bei der Hydrierung gebildete Produkt, also das entsprechende Cyclohexanderivat als Lösungsmittel eingesetzt, gegebenenfalls neben anderen Lösungs- oder Verdünnungsmitteln. In jedem Fall kann ein Teil des im Verfahren gebildeten Produkts der noch zu hydrierenden Benzolpolycarbonsäure oder des Derivats davon beigemischt werden. Bezogen auf das Gewicht der zur Hydrierung vorgesehenen Verbindung wird vorzugsweise die 1- bis 30fache, besonders bevorzugt die 5- bis 20fache, insbesondere die 5- bis 10fache Menge des Umsetzungsproduktes als Lösungs- oder Verdünnungsmittel zugemischt.

Wie bereits oben ausgeführt, umfaßt der erfindungsgemäß verwendete Begriff *"Benzolpolycarbonsäuren oder Derivate davon"* sowohl die jeweiligen Benzolpolycarbonsäuren an sich sowie Derivate davon, wobei insbesondere Mono-, Di- oder ggf. Tri- oder Tetraester sowie Anhydride der Benzolpolycarbonsäuren zu nennen sind. Die eingesetzten Ester sind Alkyl-, Cykloalkyl- sowie Alkoxyalkylester, wobei die Alkyl-, Cycloalkyl- sowie Alkoxyalkylgruppen in der Regel 1 bis 30, vorzugsweise 2 bis 20 und besonders bevorzugt 3 bis 18 Kohlenstoffatome umfassen und verzweigt oder linear sein können.

Im einzelnen sind zu nennen:
Terephthalsäurealkylester, wie z.B. Terephthalsäuremonomethylester, Terephthalsäuredimethylester, Terephthalsäurediethylester, Terephthalsäuredi-n-propylester, Terephthalsäuredi-n-butylester, Terephthalsäuredi-tert-butylester, Terephthalsäurediisobutylester, Terephthalsäuremonoglykolester, Terephthalsäurediglykolester, Terephthalsäuredi-n-octylester, Terephthalsäurediisooctylester, Terephthalsäuremono-2-ethylhexylester, Terephthalsäuredi-2-ethylhexylester, Terephthalsäuredi-n-nonylester, Terephthalsäurediisononylester, Terephthalsäuredin-decylester, Terephthalsäuredi-n- undecylester, Terephthalsäurediisodecylester, Terephthalsäurediisododecylester, Terephthalsäuredi-n-octadecylester, Terephthalsäurediisooctadecylester, Terephthalsäuredi-n-eicosylester, Terephthalsäuremonocyclohexylester, Terephthalsäuredicyclohexylester;
Phthalsäurealkylester, wie z.B. Phthalsäuremonomethylester, Phthalsäuredimethylester, Phthalsäurediethylester, Phthalsäuredi-n-propylester, Phthalsäuredi-n-butylester, Phthalsäuredi-tert.-butylester, Phthalsäurediisobutylester, Phthalsäuremonoglykolester, Phthalsäurediglykolester, Phthalsäuredi-n-octylester, Phthalsäurediisooctylester, Phthalsäuredi-2-ethylhexylester, Phthalsäuredi-n-nonylester, Phthalsäurediisononylester, Phthalsäuredi-n-decylester, Phthalsäurediisodecylester, Phthalsäuredi-n-undecylester, Phthalsäurediisododecylester, Phthalsäuredi-noctadecylester, Phthalsäurediisooctadecylester, Phthalsäuredi-n-eicosylester, Phthalsäuremonocyclohexylester, Phthalsäuredicyclohexylester;
Isophthalsäurealkylester, wie z.B. Isophthalsäuremonomethylester, Isophthalsäuredimethylester, Isophthalsäurediethylester, Isophthalsäuredi-n-propylester, Isophthalsäuredi-n-butylester, Isophthalsäuredi-tert.-butylester, Isophthalsäurediisobutylester, Isophthalsäuremonoglykolester, Isophthalsäurediglykolester, Isophthalsäuredi-noctylester, Isophthalsäurediisooctylester, Isophthalsäuredi-2-ethylhexylester, Isophthalsäuredi-n-nonylester, Isophthalsäurediisononylester, Isophthalsäuredi-ndecylester, Isophthalsäurediisodecylester, Isophthalsäuredi-n-undecylester, Isophthalsäurediisododecylester, Isophthalsäuredi-n-octadecylester, Isophthalsäurediisooctadecylester, Isophthalsäuredi-n-eicosylester, Isophthalsäuremonocyclohexylester, Isophthalsäuredicyclohexylester.

Trimellitsäurealkylester, wie z.B. Trimellitsäuremonomethylester, Trimellitsäuredimethylester, Trimellitsäurediethylester, Trimellitsäuredi-n-propylester, Trimellitsäuredi-n-butylester, Trimellitsäuredi-tert-butylester, Trimellitsäurediisobutylester, Trimellitsäuremonoglykolester, Trimellitsäurediglykolester, Trimellitsäuredi-n-octylester, Trimellitsäurediisooctylester, Trimellitsäuredi-2-ethylhexylester, Trimellitsäuredi-n-nonylester, Trimellitsäurediisononylester, Trimellitsäuredi-n-decylester, Trimellitsäurediisodecylester, Trimellitsäuredi-n-undecylester, Trimellitsäurediisododecylester, Trimellitsäuredi-n-octadecyl- ester, Trimellitsäurediisooctadecylester, Trimellitsäuredi-n-eicosylester, Trimellitsäuremonocyclohexylester, Trimellitsäuredicyclohexylester sowie Trimellitsäuretrimethylester, Trimellitsäuretriethylester, Trimellitsäuretri-n-propylester, Trimellitsäuretri-nbutylester, Trimellitsäuretri-tert-butylester, Trimellitsäuretriisobutylester, Trimellitsäuretriglykolester, Trimellitsäuretri-n-octylester, Trimellitsäuretriisooctylester, Trimellitsäuretri-2-ethylhexylester, Trimellitsäuretri-n-nonylester, Trimellitsäuretriisododecylester, Trimellitsäuretri-n-undecylester, Trimellitsäuretriisododecylester, Trimellitsäuretri-n-octadecylester, Trimellitsäuretriisooctadecylester, Trimellitsäuretri-n-eicosylester, Trimellitsäuretricyclohexylester.

Trimesinsäurealkylester, wie z.B. Trimesinsäuremonomethylester, Trimesinsäuredimethylester, Trimesinsäurediethylester, Trimesinsäuredi-n-propylester, Trimesinsäuredi-n-butylester, Trimesinsäuredi-tert-butylester, Trimesinsäurediisobutylester, Trimesinsäuremonoglykolester, Trimesinsäurediglykolester, Trimesinsäuredi-noctylester, Trimesinsäurediisooctylester, Trimesinsäuredi-2-ethylhexylester, Trimesinsäuredi-n-nonylester, Trimesinsäurediisononylester, Trimesinsäuredi-ndecylester, Trimesinsäurediisodecylester, Trimesinsäuredi-n-undecylester, Trimesinsäurediisododecylester, Trimesinsäuredi-n-octadecylester, Trimesinsäurediisooctadecylester, Trimesinsäuredi-n-eicosylester, Trimesinsäuremonocyclohexylester, Trimesinsäuredicyclohexylester, sowie Trimesinsäuretrimethylester, Trimesinsäuretriethylester, Trimesinsäuretri-n-propylester, Trimesmsäuretri-n-butylester, Trimesinsäuretri-tert-butylester, Trimesinsäuretriisobutylester, Trimesinsäuretriglykolester, Trimesinsäuretri-n-octylester, Trimesinsäuretriisooctylester, Trimesinsäuretri-2-ethylhexylester, Trimesinsäuretri-n-nonylester, Trimesinsäuretriisododecylester, Trimesinsäuretri-n-undecylester, Trimesinsäuretriisododecylester, Trimesinsäuretri-n-octadecylester, Trimesinsäuretriisooctadecylester, Trimesinsäuretri-n-eicosylester, Trimesinsäuretricyclohexylester.

Hemimellitsäurealkylester, wie z.B. Hemimellitsäuremonomethylester, Hemimellitsäuredimethylester, Hemimellitsäurediethylester, Hemimellitsäuredi-n-propylester, Hemimellitsäuredi-n-butylester, Hemimellitsäuredi-tert-butylester, Hemimellitsäurediisobutylester, Hemimellitsäuremonoglykolester, Hemimellitsäurediglykolester, Hemimellitsäuredi-n-octylester, Hemimellitsäurediisooctylester, Hemimellitsäuredi-2-ethylhexylester, Hemimellitsäuredi-n-nonylester, Hemimellitsäurediisononylester, Hemimellitsäuredi-n-decylester, Hemimellitsäurediisodecylester, Hemimellitsäuredin-undecylester, Hemimellitsäurediisododecylester, Hemimellitsäuredi-n-octadecylester, Hemimellitsäurediisooctadecylester, Hemimellitsäuredi-n-eicosylester, Hemimellitsäuremonocyclohexylester, Hemimellitsäuredicyclohexylester, sowie Hemimellitsäuretrimethylester, Hemimellitsäuretriethylester, Hemimellitsäuretri-npropylester, Hemimellitsäuretri-n-butylester, Hemimellitsäuretri-tert-butylester, Hemimellitsäuretriisobutylester, Hemimellitsäuretriglykolester, Hemimellitsäuretrin-octylester, Hemimellitsäuretriisooctylester, Hemimellitsäuretri-2-ethylhexylester, Hemimellitsäuretri-n-nonylester, Hemimellitsäuretriisododecylester, Hemimellitsäuretri-n-undecylester, Hemimellitsäuretriisododecylester, Hemimellitsäuretri-noctadecylester, Hemimellitsäuretriisooctadecylester, Hemimellitsäuretri-n-eicosylester, Hemimellitsäuretricyclohexylester.

Pyromellitsäurealkylester, wie z.B. Pyromellitsäuremonomethylester, Pyromellitsäuredimethylester, Pyrromellitsäurediethylester, Pyromellitsäuredi-n-propylester, Pyromellitsäuredi-n-butylester, Pyromellitsäuredi-tert.-butylester, Pyromellitsäurediisobutylester, Pyromellitsäuremonoglykolester, Pyromellitsäurediglykolester, Pyromellitsäuredi-n-octylester, Pyromellitsäurediisooctylester, Pyromellitsäuredi-2-ethylhexylester, Pyromellitsäuredi-n-nonylester, Pyromellitsäurediisononylester, Pyromellitsäuredi-n-decylester, Pyromellitsäurediisodecylester, Pyromellitsäuredi-nundecylester, Pyromellitsäurediisododecylester, Pyromellitsäuredi-n-octadecylester, Pyromellitsäurediisooctadecylester, Pyromellitsäuredi-n-eicosylester, Pyromellitsäuremonocyclohexylester, Pyromellitsäuretrimethylester, Pyromellitsäurctriethylester, Pyromellitsäuretri-n-propylester, Pyromellitsäuretri-n-butylester, Pyromellitsäuretri-tert-butylester, Pyromellitsäuretriisobutylester, Pyromellitsäuretriglykolester, Pyromellitsäuretri-n-octylester, Pyromellitsäuretriisooctylester, Pyromellitsäuretri-2-ethylhexylester, Pyromellitsäuretri-n-nonylester, Pyromellitsäuretriisododecylester, Pyromellitsäuretri-n-undecylester, Pyromellitsäuretriisododecylester, Pyromellitsäuretri-n-octadecylester, Pyromellitsäuretriisooctadecylester, Pyromellitsäuretri-n-eicosylester, Pyromellitsäuretricyclohexylester, sowie Pyromellitsäuretetramethylester, Pyromellitsäuretetraethylester, Pyromellitsäuretetra-n-propylester, Pyromellitsäuretetra-n-butylester, Pyromellitsäuretetra-tert-butylester, Pyromellitsäuretetraisobutylester, Pyromellitsäuretetraglykolester, Pyromellitsäuretetra-noctylester, Pyromellitsäuretetraisooctylester, Pyromellitsäuretetra-2-ethylhexylester, Pyromellitsäuretetra-n-nonylester, Pyromellitsäuretetraisododecylester, Pyromellitsäuretetra-n-undecylester, Pyromellitsäuretetraisododecylester, Pyromellitsäuretetran-octadecylester, Pyromellitsäuretetraisooctadecylester, Pyromellitsäuretetra-neicosylester, Pyromellitsäuretetracyclohexylester.

Anhydride der Phthalsäure, Trimellitsäure, Hemimellitsäure und Pyromellitsäure.

Selbstverständlich können auch Gemische aus zwei oder mehr dieser Verbindungen-eingesetzt werden.

Bei den erfindungsgemäß erhaltenen Produkten handelt es sich dabei stets um die entsprechenden Cyclohexanpolycarbonsäuren oder Cyclohexanpolycarbonsäurederivate.

Ferner betrifft die vorliegende Erfindung die folgenden neuen Cyclohexanpolycarbonsäuren oder Cyclohexanpolycarbonsäurederivate an sich:
Cyclohexan-1,2-dicarbonsäuredi(isopentyl)ester, erhältlich durch Hydrierung von Di(isopentyl)phthalat mit der Chemical Abstracis Registry- Nummer (im folgenden: CAS Nr.) 84777-06-0:
Cyclohexan-1,2-dicarbonsäuredi(isoheptyl)ester, erhältlich durch Hydrierung von Di(isoheptyl)phthalat mit der CAS Nr. 71888-89-6;
Cyclohexan-1,2-dicarbonsäuredi(isononyl)ester, erhältlich durch Hydrierung eines Di(isononyl)phthalts mit der CAS Nr. 68515-48-0;
Cyclohexan-1,2-dicarbonsäuredi(isononyl)ester, erhältlich durch Hydrierung eines Di(isononyl)phthalats mit der CAS Nr. 28553-12-0, basierend auf n-Buten;
Cyclohexan-1,2-dicarbonsäuredi(isononyl)ester, erhältlich durch Hydrierung eines Di(isononyl)phthalats mit der CAS Nr. 28553-12-0 basierend auf Isobuten;
ein 1,2-Di-C₉-Ester der Cyclohexandicarbonsäure, erhältlich durch Hydrierung eines Di(hexyl)phthalts mit der CAS Nr. 68515-46-8;
ein Cyclohexan-1,2-dicarbonsäuredi(isodecyl)ester erhältlich durch Hydrierung eines Di(isodecyl)phthalats mit der CAS Nr. 68515-49-1;
ein 1,2-Di-C₇₋₁₁-Ester der Cyclohexandicarbonsäure, erhältlich durch Hydrierung des entsprechenden Phthalsäureesters mit der CAS Nr. 68515-42-4;
ein 1,2-Di-C₇₋₁₁-Ester der Cyclohexandicarbonsäure, erhältlich durch Hydrierung der Di-C₇₋₁₁-Phthalate mit folgenden CAS Nr.
111 381-89-6,
111 381 90-9,
111 381 91-0,
68515-44-6,
68515-45-7 und
3648-20-7;
ein 1,2-Di-C₉₋₁₁-Ester der Cyclohexandicarbonsäure, erhältlich durch Hydrierung eines Di-C₉₋₁₁-Phthalats mit der CAS Nr. 98515-43-5;
ein 1,2-Di(isodecyl)cyclohexandicarbonsäureester, erhältlich durch Hydrierung eines Di(isodecyl)phthalats, das hauptsächlich aus Di-(2-propylheptyl)phthalt besteht;
ein 1,2-Di-C₇₋₉-Cyclohexandicarbonsäureester, erhältlich durch Hydrierung des entsprechenden Phthalsäureesters, der verzweigtkettige oder lineare C₇₋₉-Alkylestergruppen aufweist; entsprechende beispielsweise als Ausgangsprodukte verwendbare Phthalsäureester haben die folgende CAS Nr.:
Di-C_{7.9}-Alkylphthalat mit der CAS Nr. 111 381-89-6;
Di-C₇-Alkylphthalat mit der CAS Nr. 68515-44-6; und
Di-C₉-Alkylphthalat mit der CAS Nr. 68515-45-7,
wobei die Benzolpolycarbonsäure oder das Derivat davon oder das Gemisch aus zwei oder mehr davon in Kontakt gebracht wird mit einem Wasserstoff enthaltenden Gas in Gegenwart eines Katalysators, der als Aktivmetall mindestens ein Metall der VIII. Nebengruppe des Periodensystems alleine oder zusammen mit mindestens einem Metall der L oder VII. Nebengruppe des Periodensystems, aufgebracht auf einem Träger, umfaßt, dadurch gekennzeichnet, dass der Träger Makroporen mit einem Porendurchmesser oberhalb von 50 nm gemäß der Definition in Pure Applied Chemistry, 45, S. 79 (1976) aufweist,
und
dass der Katalysator als Aktivmetall mindestens ein Metall der VIII. Nebengruppe des Periodensystems alleine oder zusammen mit mindestens einem Metall der L oder VII. Nebengruppe des Periodensystems in einer Menge von 0,01 bis 30 Gew.-%, bezogen auf das Gesamtgewicht des Katalysators, aufgebracht auf einem Träger, umfaßt, wobei 5 bis 50% des Porenvolumens des Trägers von Makroporen mit einem Porendurchmesser im Bereich von 50 nm bis 10.000 mit und 50 bis 95% des Porenvolumens des Trägers von Mesoporen mit einem Porendurchmesser im Bereich von 2 bis 50 nm gebildet werden, wobei sich die Summe der Anteile Porenvolumina zu 100% addiert.

Darüberhinaus betrifft die vorliegende Erfindung auch die Verwendung der erfindungsgemäßen Cyclonexanpolycarbonsäureestern, insbesondere der mit dem erfindungsgemäßen Verfahren erhaltenen Cyclohexanpolycarbonsäureester als Weichmacher in Kunststoffen, wobei hier allgemein Diester und Triester mit Alkylgruppen mit 3 bis 18 Kohlenstoffatomen bevorzugt und die oben genannten, individuell aufgeführten Ester mit 3 bis 18 Kohlenstoffatomen insbesondere bevorzugt sind.

Weiter bevorzugt werden die oben explizit aufgeführten neuen C₅-, C₇-, C₉-, C₁₀-, C₇₋₁₁-, C₉₋₁₁- und C₇₋₉-Ester der 1,2-Cyclohexandicarbonsäure, die durch erfindungsgemäße Hydrierung der entsprechenden Phthalate erhältlich sind und weiter bevorzugt die Hydrierungsprodukte der kommerziell erhältlichen Benzolcarbonsäureester mit den Handelsnamen Jayflex DINP (CAS Nr. 68515-48-0), Jayflex DIDP (CAS Nr. 6851549-1), Palatinol 9-P, Vestinol 9 (CAS Nr. 28553-12-0), TOTM-I (CAS Nr. 3319-31-1), Linplast 68-TM und Palatinol N (CAS Nr. 28553-12-0) als Weichmacher in Kunststoffen eingesetzt. Darunter bevorzugt ist wiederum die Verwendung dieser Verbindungen bzw. von Gemische daraus als Weichmacher in Massenkunststoffen, wie z.B. PVC, PVB, sowie PVAc.

Verglichen mit den bislang hauptsächlich als Weichmacher verwendeten Phthalaten besitzen die erfindungsgemäß verwendeten Cyclohexanpolycarbonsäure(derivate) eine niedrigere Dichte und Viskosität und führen u.a. zu einer Verbesserung der Kälteflexibilität des Kunststoffs gegenüber der Verwendung der entsprechenden Phthalate als Weichmacher, wobei Eigenschaften wie Shore A-Härte und mechanische Eigenschaften der resultierenden Kunststoffe identisch zu denen sind, die bei Verwendung von Phthalaten resultieren. Ferner besitzen die erfindungsgemäß verwendeten Cyclohexanpolycarbonsäure(derivate) ein besseres Verarbeitungsverhalten im Dry-Blend und als Folge eine erhöhte Produktionsgeschwindigkeit sowie in Plastisol-Verarbeitungen Vorteile durch eine deutlich niedrigere Viskosität gegenüber den entsprechenden Phthalaten.

Im folgenden soll nunmehr das erfindungsgemäße Verfahren anhand einiger Ausführungsbeispiele näher erläutert werden.

### BEISPIELE

### Herstellungsbeispiel

Ein meso-/makroporöser Aluminiumoxidträger in Form von 4 mm-Extrudaten, der eine BET-Oberfläche von 238 m²/g und ein Porenvolumen von 0,45 ml/g besaß, wurde mit einer wäßrigen Ruthenium-(III)-nitrat-Lösung, die eine Konzentration von 0,8 Gew.-% aufwies, getränkt, 0,15 ml/g (ungefähr 33% des Gesamtvolumens) der Poren des Trägers besaßen einen Durchmesser im Bereich von 50 nm bis 10.000 nm und 0,30 ml/g (ungefähr 67% des Gesamt Porenvolumens) der Poren des Trägers wiesen einen Porendurchmesser im Bereich von 2 bis 50 nm auf. Das während des Tränkens vom Träger aufgenommene Lösungsvolumen entsprach dabei in etwa dem Porenvolumen des verwendeten Trägers.

Anschließend wurde der mit der Ruthenium-(III)-nitrat-Lösung getränkte Träger bei 120 °C getrocknet und bei 200 °C im Wasserstrom aktiviert (reduziert). Der so hergestellte Katalysator enthielt 0,05 Gew.-% Ruthenium, bezogen auf das Gewicht des Katalysators.

### Beispiel 1

In einem 300 ml-Druckreaktor wurden 10 g des Ru-Katalysators gemäß Herstellungsbeispiel in einem Katalysator-Korbeinsatz vorgelegt und mit 197 g (0,5 mol) Diisooctylphthalat versetzt. Die Hydrierung wurde mit reinem Wasserstoff bei einem konstanten Druck von 200 bar und einer Temperatur von 80 °C durchgeführt. Es wurde solange hydriert, bis kein Wasserstoff mehr aufgenommen wurde (4 h). Der Reaktor wurde anschließend entspannt. Der Umsatz des Diisooctylphthalats betrug 100%. Die Ausbeute an Di- isooctylhexahydrophthalat lag bei 99,7%, bezogen auf die Gesamtmenge des eingesetzten Diisooctylphthalats.

### Beispiel 2

In einem 300 ml-Druckreaktor wurden 10 g des Ru-Katalysators in einem Katalysator-Korbeinsatz vorgelegt und mit 194 g (0,46 mol) Diisononylphthalat versetzt. Die Hydrierung wurde mit reinem Wasserstoff bei einem konstanten Druck von 100 bar und einer Temperatur von 80 °C durchgeführt. Es wurde solange hydriert, bis kein Wasserstoff mehr aufgenommen wurde (10 h). Anschließend wurde der Reaktor entspannt. Der Umsatz an Diisononylphthalat betrug 100%. Die Ausbeute an Diisononylhexahydrophthalat lag bei 99,5%, bezogen auf die Gesamtmenge des eingesetzten Diisononylphthalats.

### Beispiel 3

In einem 300 ml-Druckreaktor wurden 10 g des Ru-Katalysators gemäß Herstellungsbeispiel in einem Katalysator-Korbeinsatz vorgelegt und mit 195 g (2,3 mol) Diisododecylphthalat versetzt. Die Hydrierung wurde mit reinem Wasserstoff bei einem konstanten Druck von 200 bar und einer Temperatur von 80 °C durchgeführt. Es wurde solange hydriert, bis kein Wasserstoff mehr aufgenommen wurde (4 h). Der Reaktor wurde anschließend entspannt. Der Umsatz an Diisododecylphthalat betrug 100%. Die Ausbeute an Di- isododecylhexahydrophthalat lag bei 99,5%, bezogen auf die eingesetzte Menge an Diisododecylphthalat.

### Beispiel 4

In einem 300 ml-Druckreaktor wurden 10 g des Ru-Katalysators in einem Katalysator-Korbeinsatz vorgelegt und mit 38,4 g (0,2 mol) Isophthalsäuredimethylester, gelöst in 100 g THF, versetzt. Die Hydrierung wurde mit reinem Wasserstoff bei einem konstanten Druck von 200 bar und einer Temperatur von 80 °C durchgeführt. Es wurde solange hydriert, bis kein Wasserstoff mehr aufgenommen wurde, und der Reaktor anschließend entspannt. Der Umsatz des Isophthalsäuredimethylesters betrug 95,3%. Die Ausbeute an Hexahydro-Isophthalsäuredimethylester lag bei 95,3%.

### Beispiel 5

In einem 300 ml-Druckreaktor wurden 10 g des Ru-Katalysators in einem Katalysator-Korbeinsatz vorgelegt und mit 25,2 g (0,1 mol) Trimesinsäuretrimethylester, gelöst in 100 g THF, versetzt. Die Hydrierung wurde mit reinem Wasserstoff bei einem konstanten Druck von 200 bar und einer Temperatur von 120 °C durchgeführt. Es wurde solange hydriert, bis kein Wasserstoff mehr aufgenommen wurde, und der Reaktor anschließend entspannt. Der Umsatz des Trimesinsäuretrimethylesters betrug 97%. Die Ausbeute an Hexahydro-Trimesinsäuretrimethylester lag bei 93%.

### Beispiel 6

In einem 300 ml-Druckreaktor wurden 10 g des Ru-Katalysators in einem Katalysator-Korbeinsatz vorgelegt und mit 25,2 g (0,1 mol) Trimellitsäuretrimethylester, gelöst in 100 g THF, versetzt. Die Hydrierung wurde mit reinem Wasserstoff bei einem konstanten Druck von 200 bar und einer Temperatur von 120 °C durchgeführt. Es wurde solange hydriert, bis kein Wasserstorf mehr aufgenommen wurde, und der Reaktor anschließend entspannt. Der Umsatz des Trimellitsäuretrimethylesters betrug 35%. Die Ausbeute an Hexahydro-Trimellitsäuretrimethylester lag bei 33%.

### Beispiel 7

In einem 300 ml-Druckreaktor wurden 10 g des Ru-Katalysators in einem Katalysator-Korbeinsatz vorgelegt und mit 10,0 g (0,03 mol) Pyromellitsäuretetramethylester, gelöst in 100 g THF, versetzt. Die Hydrierung wurde mit reinem Wasserstoff bei einem konstanten Druck von 200 bar und einer Temperatur von 80 °C durchgeführt. Es wurde solange hydriert, bis kein Wasserstoff mehr aufgenommen wurde, und der Reaktor anschließend entspannt. Der Umsatz des Pyromellitsäuretetramethylesters betrug 45%. Die Ausbeute an Hexahydro-Pyromellitsäuretetramethylester lag bei 44%.

### Beispiel 8

In einem 1,2 1-Druckreaktor wurden 53 g des geträgerten Ru-Katalysators in einem Katalysator-Korbeinsatz vorgelegt und mit 800 g (1,9 mol) Jayflex DINP (CAS Nr. 68515-48-0) versetzt. Die Hydrierung wurde mit reinem Wasserstoff bei einem konstanten Druck von 200 bar und einer Temperatur vom 100°C durchgeführt. Es wurde so lange hydriert, bis kein Wasserstoff mehr aufgenommen wurde (6 h) und der Reaktor anschließend entspannt. Der Umsatz an Jayflex DINP betrug 100%. Die Ausbeute des entsprechenden Cyclohexandicarbonsäureesters lag bei 99,5%, bezogen auf die Gesamtmenge des eingesetzten Jayflex DINP.

### Beispiel 9

In einem 0,3 1-Druckreaktor wurden 10 g des geträgerten Ru-Katalysators in einem Katalysator-Korbeinsatz vorgelegt und mit 150 g (0,35 mol) Palatinol 9-P versetzt. Die Hydrierung wurde mit reinem Wasserstoff bei einem konstanten Druck von 200 bar und einer Temperatur von 120°C durchgeführt. Es wurde so lange hydriert, bis kein Wasserstoff mehr aufgenommen wurde (2 h) und der Reaktor anschließend entspannt. Der Umsatz an Palatinol 9-P betrug 100%. Die Ausbeute des entsprechenden Cyclohexandicarbonsäureesters lag bei 99,4%, bezogen auf die Gesamtmenge des eingesetzten Palatinol 9-P (1,2-Di(nonyl, linear und verzweigt)-benzoldicarbonsäureester).

### Beispiel 10

In einem 1,2 1-Druckreaktor wurden 53 g des geträgerten Ru-Katalysators in einem Katalysator-Korbeinsatz vorgelegt und mit 780 g (1,87 mol) Vestinol 9 (CAS Nr. 28553-12-0) versetzt. Die Hydrierung wurde mit reinem Wasserstoff bei einem konstanten Druck von 200 bar und einer Temperatur von 120°C durchgeführt. Es wurde so lange hydriert, bis kein Wasserstoff mehr aufgenommen wurde (4 h) und der Reaktor anschließend entspannt. Der Umsatz an Vestinol 9 betrug 100%. Die Ausbeute des entsprechenden Cyclohexandicarbonsäureesters lag bei 99,4%, bezogen auf die Gesamtmenge des eingesetzten Vestinol 9.

### Beispiel 11

In einem 1,2 1-Druckreaktor wurden 53 g des geträgerten Ru-Katalysators in einem Katalysator-Korbeinsatz vorgelegt und mit 760 g (1,7 mol) Jayflex DIDP (CAS Nr. 68515-49-1) versetzt. Die Hydrierung wurde mit reinem Wasserstoff bei einem konstanten Druck von 200 bar und einer Temperatur von 100°C durchgeführt. Es wurde so lange hydriert, bis kein Wasserstoff mehr aufgenommen wurde (10 h) und der Reaktor anschließend entspannt. Der Umsatz an Jayflex DIDP betrug 100%. Die Ausbeute des entsprechenden Cyclohexandicarbonsäureesters lag bei 99,5%, bezogen auf die Gesamtmenge des eingesetzten Jayflex DIDP.

### Beispiel 12

In einem 1,2 1-Druckreaktor wurden 53 g des geträgerten Ru-Katalysators in einem Katalysator-Korbeinsatz vorgelegt und mit 800 g (1,56 mol) TOTM-I (1,2,4-Tri(2-ethylhexyl)benzoltricarbonsäureester) versetzt. Die Hydrierung wurde mit reinem Wasserstoff bei einem konstanten Druck von 200 bar und einer Temperatur von 100°C durchgeführt. Es wurde so lange hydriert, bis kein Wasserstoff mehr aufgenommen wurde (20 h) und der Reaktor anschließend entspannt. Der Umsatz an TOTM-I betrug 95%. Die Ausbeute des entsprechenden Cyclohexandicarbonsäureesters lag bei 94%, bezogen auf die Gesamtmenge des eingesetzten TOTM-I.

### Beispiel 13

In einem 300 ml-Druckreaktor wurden 10 g des geträgerten Ru-Katalysators in einem Katalysator-Korbeinsatz vorgelegt und mit 150 g (0,32 mol) Linplast 68-TM (1,2,4-Tri(lineare C₆₋₈-alkyl)benzoltricarbonsäureester) versetzt. Die Hydrierung wurde mit reinem Wasserstoff bei einem konstanten Druck von 200 bar und einer Temperatur von 120°C durchgeführt. Es wurde so lange hydriert, bis kein Wasserstoff mehr aufgenommen wurde (11 h) und der Reaktor anschließend entspannt. Der Umsatz an Linplast 68-TM betrug 100%. Die Ausbeute des entsprechenden Cyclohexandicarbonsäureesters lag bei 99,2%, bezogen auf die Gesamtmenge des eingesetzten Linplast 68-TM.

### Beispiel 14

Ein senkrecht stehendes Hochdruckrohr aus Edelstahl mit einem inneren Durchmesser von 30 mm und einer Länge von 2,2 m wurde mit 1,4 1 des geträgerten Ru-Katalysators gefüllt. Im Fall der Sumpffahrweise wurden 0,45 kg/h Palatinol N (CAS Nr. 28553-12-0) mit reinem Wasserstoff bei einer mittleren Temperatur von 125°C und einem Druck von 200 bar von unten nach oben durch den Reaktor gepumpt. Ein Teil des Reaktionsproduktes wurde nach Verlassen des Hochdruckreaktors zusammen mit neuem Palatinol N erneut in den Reaktor gepumpt, das restliche Reaktionsprodukt in einem Auffangbehälter entspannt. Abgaskontrolliert wurde mit einem 20%-igem Überschuß des theoretisch benötigten Wasserstoffs hydriert. Die gaschromatographische Analyse des Reaktionsaustrages zeigte, daß Palatinol N zu 99,5% umgesetzt worden war. Der entsprechende Cyclohexandicarbonsäureester konnte mit einer Selektivität von 99,2% erhalten werden. Um die verbleibenden 0,5% Palatinol N aus dem Reaktionsaustrag zu entfernen, wurde dieser mit 1 kg/h von unten nach oben durch den Reaktor gepumpt und der Austrag in einen Auffangbehälter entspannt. Die Wasserstoffzudosierung wurde wie oben beschrieben beibehalten. Palatinol N konnte danach nicht mehr im Austrag nachgewiesen werden. Die Selektivität zum entsprechenden Cyclohexandicarbonsäureester betrug nach der zweiten Hydrierung 99%. Als Nebenkomponenten konnten etwa 1% Leichtsieder (Komponenten mit einem niedrigeren Siedepunkt, als der des Cyclohexandicarbonsäureester) nachgewiesen werden. Diese liesen sich mittels Wasserdampfdestillation bei 170°C und einem Druck von 50 mbar abreichern. Der Reaktionsaustrag bestand nach dieser Aufarbeitung aus 99,7% Cyclohexandicarbonsäureester.

## Patentansprüche

1. Verfahren zur Hydrierung
einer Benzolpolycarbonsäure oder eines Derivats davon oder eines Gemischs aus zwei oder mehr davon durch Inkontaktbringen der Benzolpolycarbonsäure oder des Derivats davon oder des Gemischs aus zwei oder mehr davon mit einem Wasserstoff enthaltenden Gas in Gegenwart eines Katalysators, der als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfaßt, **dadurch gekennzeichnet, dass** der Träger Makroporen mit einem Porendurchmesser oberhalb von 50 nm gemäß der Definition in Pure Applied Chemistry, 45, S. 79 (1976) aufweist,
und
dass der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt, wobei 5 bis 50% des Porenvolumens des Trägers von Makroporen mit einem Porendurchmesser im Bereich von 50 nm bis 10.000 nm und 50 bis 95% des Porenvolumens des Trägers von Mesoporen mit einem Porendurchmesser im Bereich von 2 bis 50 nm gebildet werden, wobei sich die Summe der Anteile Porenvolumina zu 100% addiert,
mit der Maßgabe, dass,
sofern Terephthalsäuredimethylester hydriert wird, die Hydrierung mit einem Katalysator, der als Aktivmetall Ruthenium alleine oder zusammen mit mindestens einem Metall der I., VII. oder VIII. Nebengruppe des Periodensystems, aufgebracht auf einem Träger, umfaßt, wobei der Träger einen mittleren Porendurchmesser von mindestens 50 nm und eine BET-Oberfläche von höchstens 30 m²/g aufweist und die Menge des Aktivmetalls 0,01 bis 30 Gew.- %, bezogen auf das Gesamtgewicht des Katalysator, beträgt, wobei das Verhältnis der Oberflächen des Aktivmetalls und des Katalysatorträgers kleiner 0,05 ist,
und/oder
eines Katalysators, der als Aktivmetall Ruthenium alleine oder zusammen mit mindestens einem Metall der I., VII. oder VIII. Nebengruppe des Periodensystems in einer Menge von 0,01 bis 30 Gew.-%, bezogen auf das Gesamtgewicht des Katalysators, aufgebracht auf einem Träger, umfaßt,
wobei 10 bis 50% des Porenvolumens des Trägers von Makroporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet werden, wobei sich die Summe der Anteile der Porenvolumina zu 100% addiert, ausgeschlossen ist

2. Verfahren nach Anspruch 1, **dadurch gekennzeichnet, dass** der Katalysator als Aktivmetall mindestens ein Metall der VIII. Nebengruppe des Periodensystems alleine oder zusammen mit mindestens einem Metall der L oder VII. Nebengruppe des Periodensystems, aufgebracht auf einem Träger, umfaßt, wobei der Träger einen mittleren Porendurchmesser von mindestens 50 nm und eine BET-Oberfläche von höchstens 30 m²/g aufweist und die Menge des Aktivmetalls 0,01 bis 30 Gew.- %, bezogen auf das Gesamtgewicht des Katalysator, beträgt.

3. Verfahren nach Anspruch 1, **dadurch gekennzeichnet, dass** der Katalysator als Aktivmetall mindestens ein Metall der VIII. Nebengruppe des Periodensystems alleine oder zusammen mit mindestens einem Metall der L oder-VII. Nebengruppe des Periodensystems in einer Menge von 0,01 bis 30 Gew.-%, bezogen auf das Gesamtgewicht des Katalysators, aufgebracht auf einem Träger, umfaßt, wobei der Träger einen mittleren Porendurchmesser von mindestens 0,1 µm, und eine BET-Oberfläche von höchstens 15 m²/g aufweist.

4. Verfahrem nach einem der Ansprüche 1 bis 3, **dadurch gekennzeichnet, dass** die Benzolpolycarbonsäure oder das Derivat davon ausgewählt wird aus der Gruppe bestehend aus Mono- und Dialkylestern der Phthalsäure, Terephthalsäure und Isophthalsäure, Mono-, Di- und Trialkylestern der Trimellitsäure, der Trimesinsäure und Hemimellitsäure, Mono-, Di-, Tri- und Tetraalkylestern der Pyrromellitsäure, wobei die Alkylgruppen linear oder verzweigt sein können und jeweils 3 bis 18 Kohlenstoffatome aufweisen, Anhydriden der Phthalsäure, Trimellitsäure und Hemimellitsäure, Pyrromellitsäuredianhydrid und Gemischen aus zwei oder mehr davon.

5. Verfahren nach einem der vorstehenden Ansprüche, **dadurch gekennzeichnet, dass** der Träger Aktivkohle, Siliciumcarbid, Aluminiumoxid, Siliciurndioxid, Titandioxid, Zirkoniumdioxid, Magnesiumoxid, Zinkoxid oder ein Gemisch aus zwei oder mehr davon enthält.

6. Verfahren nach einem der vorstehenden Ansprüche, **dadurch gekennzeichnet, dass** die Hydrierung in Gegenwart eines Lösungs- oder Verdünnungsmittels durchgeführt wird.

7. Verfahren nach einem der vorstehenden Ansprüche, **dadurch gekennzeichnet, dass** die Hydrierung kontinuierlich durchgeführt wird.

8. Cyclohexan-1,2-dicarbonsäuredi(isopentyl)ester, erhältlich durch Hydrierung von Di(isopentyl)phthalat mit der Chemical Abstracts Registry- Nummer (im folgenden: CAS Nr.) 84777-06-0;
Cyclohexan-1,2-dicarbonsäuredi(isoheptyl)ester, erhältlich durch Hydrierung von Di(isoheptyl)phthalat mit der CAS Nr. 71888-89-6;
Cyclohexan-1,2-dicarbonsäuredi(isononyl)ester, erhältlich durch Hydrierung eines Di(isononyl)phthalts mit der CAS Nr. 68515-48-0;
Cyclohexan-1,2-dicarbonsäuredi(isononyl)ester, erhältlich durch Hydrierung eines Di(isononyl)phthalats mit der CAS Nr. 28553-12-0, basierend auf n-Buten;
Cyclohexan-1,2-dicarbonsäuredi(isononyl)ester, erhältlich durch Hydrierung eines Di(isononyl)phthalats mit der CAS Nr. 28553-12-0, basierend auf Isobuten;
ein 1,2-Di-C₉-Ester der Cyclohexandicarbonsäure, erhältlich durch Hydrierung eines Di(hexyl)phthalts mit der CAS Nr. 68515-46-8;
ein Cyclohexan-1,2-dicarbonsäuredi(isodecyl)ester erhältlich durch Hydrierung eines Di(isodecyl)phthalats mit der CAS Nr. 68515-49-1;
ein 1,2-Di-C₇₋₁₁-Ester der Cyclohexandicarbonsäure, erhältlich durch Hydrierung des entsprechenden Phthalsäureesters mit der CAS Nr. 68515-42-4;
ein 1,2-Di-C₇₋₁₁-Ester der Cyclohexandicarbonsäure, erhältlich durch Hydrierung der Di-C₇₋₁₁-Phthalate mit folgenden CAS Nr.:
111 381-89-6,
111 381 90-9,
111 381 91-0,
68515-44-6,
68515-45-7 und
3648-20-7;
ein 1,2-Di-C₉₋₁₁-Ester der Cyclohexandicarbonsäure, erhältlich durch Hydrierung eines Di-C₉₋₁₁-Phthalats mit der CAS Nr. 98515-43-5;
ein 1,2-Di(isodecyl)cyclohexandicarbonsäureester, erhältlich durch Hydrierung eines Di(isodecyl)phthalats, das hauptsächlich aus Di-(2-propylheptyl)phthalt besteht;
ein 1,2-Di-C₇₋₉-Cyclohexandicarbonsäureester, erhältlich durch Hydrierung des entsprechenden Phthalsäureesters, der verzweigtkettige oder lineare C₇₋₉-Alkylestergruppen aufweist,
wobei die Benzolpolycarbonsäure oder das Derivat davon oder das Gemisch aus zwei oder mehr davon in Kontakt gebracht wird mit einem Wasserstoff enthaltenden Gas in Gegenwart eines Katalysators, der als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfaßt, **dadurch gekennzeichnet, dass** der Träger Makroporen mit einem Porendurchmesser oberhalb von 50 nm gemäß der Definition in Pure Applied Chemistry, 45, S. 79 (1976) aufweist,
und
dass der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt, wobei 5 bis 50% des Porenvolumens des Trägers von Makroporen mit einem Porendurchmesser im Bereich von 50 nm bis 10.000 nm und 50 bis 95% des Porenvolumens des Trägers von Mesoporen mit einem Porendurchmesser im Bereich von 2 bis 50 nm gebildet werden, wobei sich die Summe der Anteile Porenvolumina zu 100% addiert.

9. Cyclohexanpolycarbonsäurederivat, ausgewählt aus der Gruppe bestehend aus:
Cyclohexan-1,2-dicarbonsäuredi(isononyl)ester, erhältlich durch Hydrierung eines Di(isononyl)phthalats mit der CAS Nr. 28553-12-0, basierend auf n-Buten;
Cyclohexan-1,2-dicarbonsäuredi(isononyl)ester, erhältlich durch Hydrierung eines Di(isononyl)phthalats mit der CAS Nr. 28553-12-0, basierend auf Isobuten,
wobei die Benzolpolycarbonsäure oder das Derivat davon oder das Gemisch aus zwei oder mehr davon in Kontakt gebracht wird mit einem Wasserstoff enthaltenden Gas in Gegenwart eines Katalysators, der als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfaßt, **dadurch gekennzeichnet, dass** der Träger Makroporen mit einem Porendurchmesser oberhalb von 50 nm gemäß der Definition in Pure Applied Chemistry, 45, S. 79 (1976) aufweist,
und
dass der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt, wobei 5 bis 50% des Porenvolumens des Trägers von Makroporen mit einem Porendurchmesser im Bereich von 50 nm bis 10.000 nm und 50 bis 95% des Porenvolumens des Trägers von Mesoporen mit einem Porendurchmesser im Bereich von 2 bis 50 nm gebildet werden, wobei sich die Summe der Anteile Porenvolumina zu 100% addiert.

10. Verwendung eines Cyclohexandicarbonsäureesters gemäß Anspruch 8 oder 9 oder eines Cyclohexantricarbonsäureesters oder eines Gemisches aus zwei oder mehr davon als Weichmacher in PVC.

11. Verwendung eines Cyclohexandicarbonsäureesters gemäß Anspruch 8 oder 9 oder eines Cyclohexantricarbonsäureesters oder eines Gemisches aus zwei oder mehr davon als Weichmacher in PVB.

12. Verwendung eines Cyclohexandicarbonsäureesters gemäß Anspruch 9 oder eines Cyclohexantricarbonsäureesters oder eines Gemisches aus zwei oder mehr davon als Weichmacher in Kunststoffen.

13. Kunststoffzusammensetzung enthaltend einen Cyclohexandicarbonsäureester gemäß Anspruch 8 oder einen Cyclohexantricarbonsäureester oder eine Gemisch aus zwei oder mehr davon, **dadurch gekennzeichnet, dass** der Kunststoff PVC ist.

14. Kunststoffzusammensetzung enthaltend einen Cyclohexandicarbonsäureester gemäß Anspruch 9 oder eine Gemisch aus zwei oder mehr davon, **dadurch gekennzeichnet, dass** der Kunststoff PVC ist.

15. Gemisch aus PVC und einem Weichmacher, **dadurch gekennzeichnet, dass** der Weichmacher ein Cyclohexandicarbonsäureester gemäß Anspruch 8 oder ein Cyclohexantricarbonsäureester oder ein Gemisch aus zwei oder mehr davon ist.

16. Gemisch aus PVC und einem Weichmacher, **dadurch gekennzeichnet, dass** der Weichmacher ein Cyclohexandicarbonsäureester gemäß Anspruch 9 oder ein Gemisch aus zwei oder mehr davon ist.

## Claims

1. A process for hydrogenating a benzenepolycarboxylic acid or a derivative thereof or a mixture of two or more thereof by bringing the benzenepolycarboxylic acid or the derivative thereof or the mixture of two or more thereof into contact with a hydrogen-containing gas in the presence of a catalyst which comprises as active metal at least one metal of transition group VIII of the Periodic Table either alone or together with at least one metal of transition group I or VII of the Periodic Table applied to a support, wherein the support contains macropores having a pore diameter above 50 nm as defined in Pure Applied Chemistry, 45, p. 79 (1976), and
wherein the catalyst comprises as active metal at least one metal of transition group VIII of the Periodic Table either alone or together with at least one metal of transition group I or VII of the Periodic Table in an amount of from 0.01 to 30% by weight, based on the total weight of the catalyst, applied to a support, where from 5 to 50% of the pore volume of the support is formed by macropores having a pore diameter in the range from 50 nm to 10,000 nm and from 50 to 95% of the pore volume of the support is formed by mesopores having a pore diameter in the range from 2 to 50 nm, where the sum of the pore volumes adds up to 100%,
with the proviso that
if the benzenepolycarboxylic ester is dimethyl terephthalate, the hydrogenation using a catalyst which comprises as active metal ruthenium either alone or together with at least one metal of transition group I, VII or VIII of the Periodic Table applied to a support, where the support has a mean pore diameter of at least 50 nm and a BET surface area of at most 30 m²/g and the amount of the active metal is from 0.01 to 30% by weight, based on the total weight of the catalyst, and the ratio of the surface areas of the active metal and the catalyst support is less than 0.05,
and/or
a catalyst which comprises as active metal ruthenium either alone or together with at least one metal of transition group I, VII or VIII of the Periodic Table in an amount of from 0.01 to 30% by weight, based on the total weight of the catalyst, applied to a support, where from 10 to 50% of the pore volume of the support is formed by macropores having a pore diameter in the range from 50 nm to 10,000 nm and from 50 to 90% of the pore volume of the support is formed by mesopores having a pore diameter in the range from 2 to 50 nm, where the sum of the pore volumes adds up to 100%, is excluded.

2. A process as claimed in claim 1, wherein the catalyst comprises as active metal at least one metal of transition group VIII of the Periodic Table either alone or together with at least one metal of transition group I or VII of the Periodic Table applied to a support, where the support has a mean pore diameter of at least 50 nm and a BET surface area of at most 30 m²/g and the amount of the active metal is from 0.01 to 30% by weight, based on the total weight of the catalyst.

3. A process as claimed in claim 1, wherein the catalyst comprises as active metal at least one metal of transition group VIII of the Periodic Table either alone or together with at least one metal of transition group I or VII of the Periodic Table in an amount of from 0.01 to 30% by weight, based on the total weight of the catalyst, applied to a support, where the support has a mean pore diameter of at least 0.1 □m and a BET surface area of at most 15 m²/g.

4. A process as claimed in any of claims 1 to 3, wherein the benzenepolycarboxylic acid or the derivative thereof is selected from the group consisting of mono- and dialkyl phthalates, mono- and dialkyl terephthalates, mono- and dialkyl isophthalates, mono-, di- and trialkyl trimellitates, mono-, di- and trialkyl trimesates, mono-, di- and trialkyl hemimellitates, mono-, di-, tri- and tetraalkyl pyromellitates, where the alkyl groups can be linear or branched and each have from 3 to 18 carbon atoms, anhydrides of phthalic acid, trimellitic acid and hemimellitic acid, pyromellitic anhydride and mixtures of two or more thereof.

5. A process as claimed in any of the preceding claims, wherein the support comprises activated carbon, silicon carbide, aluminum oxide, silicon dioxide, titanium dioxide, zirconium dioxide, magnesium oxide, zinc oxide or a mixture of two or more thereof.

6. A process as claimed in any of the preceding claims, wherein the hydrogenation is carried out in the presence of a solvent or diluent.

7. A process as claimed in any of the preceding claims, wherein the hydrogenation is carried out continuously.

8. Di(isopentyl) cyclohexane-1,2-dicarboxylate, obtainable by hydrogenatng di(isopentyl) phthalate having the Chemical Abstracts Registry Number (hereinbelow: CAS no.) 84777-06-0;
di(isoheptyl) cyclohexane-1,2-dicarboxylate, obtainable by hydrogenating di(isoheptyl) phthalate having the CAS no. 71888-89-6;
di(isononyl) cyclohexane-1,2-dicarboxylate obtainable by hydrogenation of di(isononyl) phthalate having the CAS no. 68515-48-0;
di(isononyl) cyclohexane-1,2-dicarboxylate, obtainable by hydrogenating di(isononyl) phthalate having the CAS no. 28553-12-0, based on n-butane;
di(isononyl) cyclohexane-1,2-dicarboxylate obtainable by hydrogenating di(isononyl) phthalate having the CAS no. 28553-12-0, based on isobutane;
a di-C₆ ester of cyclohexane-1,2-dicarboxylic acid obtainable by hydrogenating a di(hexyl) phthalate having the CAS no. 68515-46-8;
di(isodecyl) cyclohexane-1,2-dicarboxylate obtainable by hydrogenating a di(isodecyl) phthalate having the CAS no. 68515-49-1;
a di-C₇₋₁₁ ester of cyclohexane-1,2-dicarboxylic acid, obtainable by hydrogenating the corresponding phthalic ester having the CAS no. 65815-42-4;
a di-C₇₋₁₁ ester of cyclohexane-1,2-dicarboxylic acid, obtainable by hydrogenating the di-C₇₋₁₁ phthalates having the following CAS nos.:
111 381-89-6,
111 381 90-9,
111 381 91-0,
68515-44-6,
68515-45-7 and
3648-20-7;
a di-C₉₋₁₁ ester of cyclohexane-1,2-dicarboxylic acid, obtainable by hydrogenating a di-C₉₋₁₁ phthalate having the CAS no. 98515-43-5;
di(isodecyl) cyclohexane-1,2-dicarboxylate, obtainable by hydrogenating a di(isodecyl) phthalate which consists mainly of di(2-propylheptyl) phthalate;
a di-C₇₋₉-ester of cyclohexane-1,2-dicarboxylic acid, obtainable by hydrogenating the corresponding phthalic ester which has branched-chain or linear C₇₋₉-alkyl ester groups, by bringing the benzenepolycarboxylic acid or the derivative thereof or the mixture of two or more thereof into contact with a hydrogen-containing gas in the presence of a catalyst which comprises as active metal at least one metal of transition group VIII of the Periodic Table either alone or together with at least one metal of transition group I or VII of the Periodic Table applied to a support, wherein the support contains macropores having a pore diameter above 50 nm as defined in Pure Applied Chemistry, 45, p. 79 (1976), and
wherein the catalyst comprises as active metal at least one metal of transition group VIII of the Periodic Table either alone or together with at least one metal of transition group I or VII of the Periodic Table in an amount of from 0.01 to 30% by weight, based on the total weight of the catalyst, applied to a support, where from 5 to 50% of the pore volume of the support is formed by macropores having a pore diameter in the range from 50 nm to 10,000 nm and from 50 to 95% of the pore volume of the support is formed by mesopores having a pore diameter in the range from 2 to 50 nm, where the sum of the pore volumes adds up to 100%.

9. A cyclohexanepolycarboxylic acid derivative selected from the group consisting of:
di(isononyl) cyclohexane-1,2-dicarboxylate obtainable by hydrogenating a di(isononyl) phthalate having the CAS no. 28553-12-0, based on n-butane;
di(isononyl) cyclohexane-1,2-dicarboxylate obtainable by hydrogenation of di(isononyl) phthalate having the CAS no. 28553-12-0, based on isobutane, by bringing the benzenepolycarboxylic acid or the derivative thereof or the mixture of two or more thereof into contact with a hydrogen-containing gas in the presence of a catalyst which comprises as active metal at least one metal of transition group VIII of the Periodic Table either alone or together with at least one metal of transition group I or VII of the Periodic Table applied to a support, wherein the support contains macropores having a pore diameter above 50 nm as defined in Pure Applied Chemistry, 45, p. 79 (1976), and
wherein the catalyst comprises as active metal at least one metal of transition group VIII of the Periodic Table either alone or together with at least one metal of transition group I or VII of the Periodic Table in an amount of from 0.01 to 30% by weight, based on the total weight of the catalyst, applied to a support, where from 5 to 50% of the pore volume of the support is formed by macropores having a pore diameter in the range from 50 nm to 10,000 nm and from 50 to 95% of the pore volume of the support is formed by mesopores having a pore diameter in the range from 2 to 50 nm, where the sum of the pore volumes adds up to 100%.

10. The use of a cyclohexanedicarboxylic ester as claimed in claim 8 or 9 or of a cyclohexanetricarboxylic ester or of a mixture of two or more thereof as plasticizer in PVC.

11. The use of a cyclohexanedicarboxylic ester as claimed in claim 8 or 9 or a cyclohexanetricarboxylic ester or a mixture of two or more thereof as plasticizer in PVB.

12. The use of a cyclohexanedicarboxylic ester as claimed in claim 9 or a cyclohexanetricarboxylic ester or a mixture of two or more thereof as plasticizer in plastics.

13. A plastic composition comprising a cyclohexanedicarboxylic ester as claimed in claim 8 or a cyclohexanetricarboxylic ester or a mixture of two or more thereof, wherein the plastic is PVC.

14. A plastic composition comprising a cyclohexanedicarboxylic ester as claimed in claim 9 or a mixture of two or more thereof, wherein the plastic is PVC.

15. A mixture of PVC and a plasticizer, wherein the plasticizer is a cyclohexanedicarboxylic ester as claimed in claim 8 or a cyclohexanetricarboxylic ester or a mixture of two or more thereof.

16. A mixture of PVC and a plasticizer, wherein the plasticizer is a cyclohexanedicarboxylic ester as claimed in claim 9 or a mixture of two or more thereof.

## Revendications

1. Procédé d'hydrogénation d'un acide benzènepolycarboxylique ou d'un de ses dérivés ou d'un mélange de deux ou de plusieurs d'entre eux par mise en contact de l'acide benzènepolycarboxylique ou de son dérivé ou du mélange de deux ou de plusieurs d'entre eux avec un gaz contenant de l'hydrogène, en présence d'un catalyseur qui comporte, appliqué sur un support, comme métal actif, au moins un métal du groupe secondaire VIII du Système Périodique, seul ou conjointement à au moins un métal du groupe secondaire I ou VII du Système Périodique,
**caractérisé en ce que** le support présente des macropores ayant un diamètre de pore supérieur à 50 nm conformément à la définition de Pure Applied Chemistry, 45, p. 79 (1976), et
**en ce que** le catalyseur comporte, appliqué sur un support, comme métal actif, au moins un métal du groupe secondaire VIII du Système Périodique, seul ou conjointement à au moins un métal du groupe secondaire I ou VII du Système Périodique, en une quantité de 0,01 à 30% en poids par rapport au poids global du catalyseur, 5 à 50% du volume poreux du support étant formés par des macropores ayant un diamètre de pore de l'ordre de 50 nm à 10.000 nm et 50 à 95% du volume poreux du support étant formés par des mésopores ayant un diamètre de pore de l'ordre de 2 à 50 nm, la somme des fractions des volumes poreux donnant 100%,
à la condition que,
dans la mesure où du téréphtalate de diméthyle est hydrogéné, l'hydrogénation est exclue avec un catalyseur qui comporte, appliqué sur un support, comme métal actif, du ruthénium seul ou conjointement à au moins un métal du groupe secondaire I, VII ou VIII du Système Périodique, le support présentant un diamètre de pore moyen d'au moins 50 nm et une surface spécifique BET d'au maximum 30 m²/g et la quantité de métal actif étant de 0,01 à 30% en poids, par rapport au poids global du catalyseur, le rapport entre les surfaces spécifiques du métal actif et du support de catalyseur étant inférieur à 0,05,
et/ou
avec un catalyseur qui comporte, appliqué sur un support, comme métal actif, du ruthénium seul ou conjointement à au moins un métal du groupe secondaire I, VII ou VIII du Système Périodique, en une quantité de 0,01 à 30% en poids par rapport au poids global du catalyseur, 10 à 50% du volume poreux du support étant formés par des macropores ayant un diamètre de pore de l'ordre de 50 nm à 10.000 nm et 50 à 90% du volume poreux du support étant formés par des mésopores ayant un diamètre de pore de l'ordre de 2 à 50 nm, la somme des fractions des volumes poreux donnant 100%.

2. Procédé suivant la revendication 1, **caractérisé en ce que** le catalyseur comporte, appliqué sur un support, comme métal actif, au moins un métal du groupe secondaire VIII du Système Périodique seul ou conjointement avec au moins un métal du groupe secondaire I ou VII du Système Périodique, le support présentant un diamètre moyen de pore d'au moins 50 nm et une surface spécifique BET d'au maximum 30 m²/g et la quantité du métal actif étant de 0,01 à 30% en poids par rapport au poids total du catalyseur.

3. Procédé suivant la revendication 1, **caractérisé en ce que** le catalyseur comporte, appliqué sur un support, comme métal actif, au moins un métal du groupe secondaire VIII du Système Périodique seul ou conjointement à au moins un métal du groupe secondaire I ou VII du Système Périodique en une quantité de 0,01 à 30% en poids, par rapport au poids total du catalyseur, le support présentant un diamètre de pore moyen d'au moins 0,1 µm et une surface spécifique BET d'au maximum 15 m²/g.

4. Procédé suivant l'une des revendications 1 à 3, **caractérisé en ce que** l'acide benzènepolycarboxylique ou son dérivé sont choisis parmi le groupe constitué des esters monoalkyliques et dialkyliques de l'acide phtalique, de l'acide téréphtalique et de l'acide isophtalique, des esters monoalkyliques, dialkyliques et trialkyliques de l'acide trimellitique, de l'acide trimésique et de l'acide hemimellitique, des esters monoalkyliques, dialkyliques, trialkyliques et tétraalkyliques de l'acide pyrromellitique, les groupes alkyle pouvant être linéaires ou ramifiés et présenter chacun 3 à 18 atomes de carbone, des anhydrides de l'acide phtalique, de l'acide trimellitique et de l'acide hemimellitique, du dianhydride d'acide pyrromellitique et des mélanges de deux ou de plusieurs de ces substances.

5. Procédé suivant l'une des revendications précédentes, **caractérisé en ce que** le support contient du charbon actif, du carbure de silicium, de l'oxyde d'aluminium, du dioxyde de silicium, du dioxyde de titane, du dioxyde de zirconium, de l'oxyde de magnésium, de l'oxyde de zinc et un mélange de deux ou de plusieurs de ces substances.

6. Procédé suivant l'une des revendications précédentes, **caractérisé en ce que** l'hydrogénation est effectuée en présence d'un solvant ou d'un diluant.

7. Procédé suivant l'une des revendications précédentes, **caractérisé en ce que** l'hydrogénation est effectuée en continu.

8. Cyclohexane-1,2-dicarboxylate de di(isopentyle), que l'on peut obtenir par hydrogénation de phtalate de di(isopentyle) ayant le numéro de registre des Chemical Abstracts (dans la suite : CAS Nr) 84777-06-0,
cyclohexane-1,2-dicarboxylate de di(isoheptyle), que l'on peut obtenir par hydrogénation de phtalate de di(isoheptyle) ayant le CAS Nr 71888-89-6,
cyclohexane-1,2-dicarboxylate de di(isononyle), que l'on peut obtenir par hydrogénation d'un phtalate de di(isononyle) ayant le CAS Nr 68515-48-0,
cyclohexane-1,2-dicarboxylate de di(isononyle), que l'on peut obtenir par hydrogénation d'un phtalate de di(isononyle) ayant le CAS Nr 28553-12-0, en se basant sur du n-butène,
cyclohexane-1,2-dicarboxylate de di(isononyle), que l'on peut obtenir par hydrogénation d'un phtalate de di(isononyle) ayant le CAS Nr 28553-12-0, en se basant sur de l'isobutène,
un 1,2-di-C₆-ester d'acide cyclohexanedicarboxylique, que l'on peut obtenir par hydrogénation d'un phtalate de di(hexyle) ayant le CAS Nr 68515-46-8,
un cyclohexane-1,2-dicarboxylate de di(isodécyle), que l'on peut obtenir par hydrogénation d'un phtalate de di(isodécyle) ayant le CAS Nr 68515-49-1,
un 1,2-di-C₇-C₁₁-ester d'acide cyclohexanedicarboxylique, que l'on peut obtenir par hydrogénation de l'ester correspondant d'acide phtalique ayant le CAS Nr 68515-42-4,
un 1,2-di-C₇-C₁₁-ester d'acide cyclohexanedicarboxylique, que l'on peut obtenir par hydrogénation des di-C₇-C₁₁-phtalates ayant les CAS Nr suivants :
111 381-89-6,
111 381 90-9,
111 381 91-0,
68515-44-6,
6851545-7, et
3648-20-7,
un 1,2-di-C₉-C₁₁-ester d'acide cyclohexanedicarboxylique, que l'on peut obtenir par hydrogénation d'un di-C₉-C₁₁-phtalate ayant le CAS Nr 98515-43-5,
un ester 1,2-di(isodécylique) d'acide cyclohexanedicarboxylique, que l'on peut obtenir par hydrogénation d'un phtalate de di(iso-décyle) qui est principalement constitué de phtalate de di(2-propylheptyle),
un 1,2-di-C₇-C₉-ester d'acide cyclohexanedicarboxylique, que l'on peut obtenir par hydrogénation de l'ester correspondant d'acide phtalique, qui présente des groupes ester alkyliques en C₇-C₉ ramifiés ou linéaires,
l'acide benzènepolycarboxylique et son dérivé ou le mélange de deux ou de plusieurs d'entre eux étant amenés en contact avec un gaz contenant de l'hydrogène en présence d'un catalyseur, qui comporte, appliqué sur un support, comme métal actif, au moins un métal du groupe secondaire VIII du Système Périodique seul ou conjointement à au moins un métal du groupe secondaire I ou VII du Système Périodique,
avec pour caractéristique que le support présente des macropores ayant un diamètre de pore supérieur à 50 nm conformément à la définition de Pure Applied Chemistry, 45, p. 79 (1976),
et
que le catalyseur comporte, appliqué sur un support, comme métal actif, au moins un métal du groupe secondaire VIII du Système Périodique seul ou conjointement à au moins un métal du groupe secondaire I ou VII du Système Périodique en une quantité de 0,01 à 30% en poids, par rapport au poids total du catalyseur, 5 à 50% du volume poreux du support étant formés par des macropores ayant un diamètre de pore de l'ordre de 50 nm jusqu'à 10.000 nm et 50 à 95% du volume poreux du support étant formés par des mésopores ayant un diamètre de pore de l'ordre de 2 à 50 nm, la somme des fractions des volumes poreux donnant 100%.

9. Dérivé d'acide cyclohexanepolycarboxylique, choisi parmi le groupe constitué :
du cyclohexane-1,2-dicarboxylate de di(isononyle), que l'on peut obtenir par hydrogénation d'un phtalate de di(isononyle) ayant le CAS Nr 28553-12-0, en se basant sur du n-butène,
du cyclohexane-1,2-dicarboxylate de di(isononyle), que l'on peut obtenir par hydrogénation d'un phtalate de di(isononyle) ayant le CAS Nr 28553-12-0, en se basant sur de l'isobutène,
l'acide benzènepolycarboxylique ou son dérivé ou le mélange de deux ou de plusieurs d'entre eux étant amené en contact avec un gaz contenant de l'hydrogène en présence d'un catalyseur, qui comporte, appliqué sur un support, comme métal actif, au moins un métal du groupe secondaire VIII du Système Périodique seul ou conjointement à au moins un métal du groupe secondaire I ou VII du Système Périodique,
avec pour caractéristique que le support présente des macropores ayant un diamètre de pore supérieur à 50 nm conformément à la définition de Pure Applied Chemistry, 45, p. 79 (1976), et
que le catalyseur comporte, appliqué sur un support, comme métal actif, au moins un métal du groupe secondaire VIII du Système Périodique seul ou conjointement à au moins un métal du groupe secondaire I ou VII du Système Périodique en une quantité de 0,01 à 30% en poids, par rapport au poids total du catalyseur, 5 à 50% du volume poreux du support étant formés par des macropores ayant un diamètre de pore de l'ordre de 50 nm à 10.000 nm et 50 à 95% du volume poreux du support étant formés par de mésopores ayant un diamètre de pore de l'ordre de 2 à 50 nm, la somme des fractions des volumes poreux donnant 100%.

10. Utilisation d'un ester d'acide cyclohexanedicarboxylique suivant l'une des revendications 8 et 9 ou d'un ester d'acide cyclohexanetricarboxylique ou d'un mélange de deux ou de plusieurs d'entre eux, comme agent ramollissant dans du PVC.

11. Utilisation d'un ester d'acide cyclohexanedicarboxylique suivant l'une des revendications 8 et 9 ou d'un ester d'acide cyclohexanetricarboxylique ou d'un mélange de deux ou de plusieurs d'entre eux comme agent ramollissant dans du PVB.

12. Utilisation d'un ester d'acide cyclohexanedicarboxylique suivant la revendication 9 ou d'un ester d'acide cyclohexanetricarboxylique ou d'un mélange de deux ou de plusieurs d'entre eux comme agent ramollissant dans des matières synthétiques.

13. Composition de matière synthétique contenant un ester d'acide cyclohexanedicarboxylique suivant la revendication 8 ou un ester d'acide cyclohexanetricarboxylique ou un mélange de deux ou de plusieurs d'entre eux, **caractérisée en ce que** la matière synthétique est du PVC.

14. Composition de matière synthétique contenant un ester d'acide cyclohexanedicarboxylique suivant la revendication 9 et un mélange de deux ou de plusieurs d'entre eux, **caractérisée en ce que** la matière synthétique est du PVC.

15. Mélange de PVC et d'un agent ramollissant, **caractérisé en ce que** l'agent ramollissant est un ester d'acide cyclohexanedicarboxylique suivant la revendication 8 ou un ester d'acide cyclohexanetricarboxylique ou un mélange de deux ou de plusieurs d'entre eux.

16. Mélange de PVC et d'un agent ramollissant, **caractérisé en ce que** l'agent ramollissant est un ester d'acide cyclohexanedicarboxylique suivant la revendication 9 ou un mélange de deux ou de plusieurs d'entre eux.
